# EUROPEAN PATENT APPLICATION

(11) **EP 2 940 136 A1**
(43) Date of publication of application: **04.11.2015**
(21) Application number: 14166712.1
(22) Date of filing: 30.04.2014
(51) Int. Cl.: C12N 15/09

(54) **Method for isolating poly(A) nucleic acids**

(71) Applicant: QIAGEN GmbH, 40724 Hilden (DE)
(72) Inventor: Christoffel, Gabriele, 40724 Hilden (DE); Schlumpberger, Martin, 40724 Hilden (DE); O'Neil, Dominic, 40724 Hilden (DE)
(74) Representative: Roth, Carla

(57) **Abstract**

The present invention pertains inter alia to a method for isolating poly(A) nucleic acids having a single stranded poly(A) stretch from a nucleic acid containing sample comprising:
(a) providing a hybridization composition comprising:
i) a nucleic acid containing sample;
ii) a hybridization solution comprising:
aa. a sodium salt;
bb. a quaternary ammonium salt;
wherein the components of the hybridization solution can be added as single solution to the sample or may be added separately in any order
to the sample;

iii) a capture probe capable of hybridizing to the poly(A) stretch of the poly(A) nucleic acids;

and incubating said hybridization composition under conditions to form nucleic acid-hybrids between the poly(A) nucleic acids and the capture probe;
(b) separating the formed hybrids from the remaining sample.

The method is in particular suitable for efficiently isolating poly(A) RNA from various samples while avoiding carry-over of unwanted non-poly(A) nucleic acids such as rRNA. Also provided are advantageous further methods, hybridization solutions and kits.

## Description

### FIELD OF INVENTION

The present invention provides a method for isolating poly(A) nucleic acids from a nucleic acids containing sample. The described method efficiently enriches poly(A) nucleic acids such as poly(A) RNA while depleting unwanted non-poly(A) nucleic acids such as e.g. rRNA. The method is particularly suitable for preparing poly(A) RNA for next generation sequencing (NGS) applications. Furthermore, compositions and kits suitable for performing the method according to the present invention are provided.

### BACKGROUND OF THE INVENTION

The present disclosure pertains to the isolation of polyadenylated nucleic acids, in particular poly(A) RNA. Polyadenylation commonly refers to the addition of a stretch consisting of multiple (usually tens to hundreds) of contiguous adenine (A) residues to a biomolecule, wherein the stretch is usually present at the 3' end of the molecule and commonly is referred to as "poly(A) tail". Nucleic acids containing a respective poly(A) stretch or poly(A) tail are commonly referred to as "poly(A) nucleic acids". In eukaryotes, polyadenylation is a process associated with the production of mature messenger RNA (mRNA) for gene expression (translation). Here, at the end of transcription (nuclear polyadenylation), a poly(A) tail is added to an RNA. In eukaryotic organisms, almost all mRNAs have a poly(A) tail at the 3' end with some minor exceptions of e.g. animal replication-dependent histone mRNAs (see e.g. Lopez et al. (RNA 14 (1): 1-10, 2007)). Furthermore, besides the vast majority of mRNAs also various eukaryotic non-coding RNAs are polyadenylated. This also includes some small RNAs, such as e.g. microRNAs which may have a poly(A) tail in their intermediary forms during microRNA maturation. Furthermore, nucleic acids can also be artificially polyadenylated to provide them with a poly(A) tail. The poly(A) tail of nucleic acids has been used as a means for isolating poly(A) nucleic acids from different sample types and in particular has been used for separating poly(A) nucleic acids from nucleic acids that lack a poly(A) tail, also referred to herein as "non-poly(A) nucleic acids".

Poly(A) nucleic acids can be isolated with the aid of a probe capable of hybridizing to the single-stranded poly(A) stretch. Such probes can capture the poly(A) nucleic acid by hybridization to the poly(A) stretch and hereinafter are also referred to as capture probe. Usually, an oligonucleotide comprising a sequence complementary to the poly(A) stretch of the poly(A) nucleic acids is used for that purpose, herein also referred to as capture oligonucleotide. To simplify the separation process of the captured poly(A) nucleic acid, often a solid support is used that is functionalized with the capture probe. The standard protocol for the specific isolation of poly(A) RNA from total RNA is based on the method of Aviv and Leder (1972). Here, short stretches of complementary DNA oligonucleotides ("oligo dT") is affixed to an insoluble matrix which is then used as a selective immobilization matrix for poly(A) RNA by setting up conditions that favor formation of RNA-DNA double strands. The total RNA sample is applied to the column in an appropriate salt buffer (originally 0.5 M KCI in 10 mM Tris, pH 7.5), encouraging hybridization of the capture probe to the poly(A) tails. The column is then subjected to extensive washing with the application buffer (containing 0.5 M KCI), then a lower-ionic-strength solution (0.1 M KCI), followed by elution of mRNA with 10 mM Tris (pH 7.5).

Subsequent modifications on this original procedure have retained the basic process of hybridization to immobilized oligo-dT, but have changed e.g. the format from columns to batch procedures to allow the procedure to be performed faster and have used NaCl or LiCl as salts. Further changes have been the replacement of cellulose with plastic or glass beads as the solid phase, and also magnetic beads have been used as solid support. This magnetic quality allows such magnetic beads to be batch isolated with the aid of a magnet. Furthermore, biotin-streptavidin linkage has been used to establish the connection between oligo-dT and the solid support, where the oligonucleotide is biotinylated and the solid support is covalently coupled to streptavidin. The hybridization can be performed in solution, linking the oligo-dT-mRNA hybrids to the solid support in a subsequent step. This procedure has tended to be an inefficient method for selectively isolating poly(A) RNA while depleting rRNA. rRNA carryover levels are often high enough to provide the same problems as presented with total RNA, especially for analysis of rare transcripts.

Other poly(A) nucleic acid isolation methods aimed at enhancing the interaction in the extended A:T hybrid by using low ionic-strength (high-stringency) washes and trying to find more "inert" materials to use as support. Isostabilizing agents, such as quaternary ammonium salts belonging to the group of tetramethylammonium (TMA+) and tetraethylammonium (TEA+) ions and the glycine amino acid derivative betaine, equalize the hydrogen bonding strength of the A:T and G:C base pairs when used at the appropriate concentrations (Jacobs et al., 1988; Jacobs et al., 1985; Gitschier et al., 1986; Melchior et al., 1973; Rees et al., 1993; Wood et al., 1985; WoZney, 1990). Such isostabilizing agents were used in the art to facilitate poly(A) isolation. US 6,812,341 describes a poly(A) enrichment method which aims at reducing rRNA carry-over during the isolation process by using isostabilizing agents such as tetramethlyammonium (TMA+) and tetraethylammonium (TEA+) ions, preferably TMAC or TEAC. WO 90/12116 relates to a generic method wherein oligo-dT -coated magnetic particles are used for the isolation of poly(A) RNA. Here, tetraalkylammonium cations are used to stabilize the A:T bonds between the poly(A) tail of the poly(A) nucleic acid and the oligo(dT) probes in combination with chaotropic salts.

Furthermore, various commercial products are available for poly(A) nucleic acid isolation such as the MagAttract® Direct mRNA M48 kit which uses oligo (dt) capture oligonucleotides immobilized on magnetic beads or the Oligotex® mRNA Kit for isolation of poly(A) RNA (Qiagen) which uses an oligo (dT) capture oligonucleotides immobilized to polystyrene-latex beads as solid support.

There is still a great demand for efficient poly(A) nucleic acid isolation methods, which efficiently capture poly(A) nucleic acids while depleting non-poly(A) nucleic acids. Such methods are e.g. needed for providing poly(A) RNA that is suitable for next generation sequencing (NGS) applications where sequencing is performed in a massively parallel manner. NGS technology platforms have in common that they require the preparation of a sequencing library which is suitable for massive parallel sequencing. Most platforms adhere to a common library preparation procedure with minor modifications before a "run" on the instrument. This procedure includes fragmenting DNA - which may be obtained from cDNA - followed by DNA repair and end polishing (blunt end or A overhang) and, finally, often platform-specific adaptor ligation. The preparation and design of such sequencing libraries is described e.g. in Voelkerding et al (Clinical Chemistry 55:4 641-658, 2009) and Metzker (Nature Reviews/ Genetics Volume 11, January 2010, pages 31-46). NGS has also been used for transcriptome sequencing. Studying whole transcriptome using next-generation sequencing technologies provides a detailed, high-throughput view of the transcriptome. Transcriptome sequencing is also referred to as RNA-sequencing (RNA-seq) and is e.g. used for mapping and quantifying transcripts in biological samples. The technique has been rapidly adopted in studies of diseases like cancer.

Preparing a sequencing library from poly(A) RNA has the advantage that RNA species, which do not carry a poly(A) tail such as rRNA (which is not of interest) are in theory not recovered and are accordingly not carried over into the sequencing reaction. Thus, most of the sequences obtained from a sequencing library that was generated using poly(A) RNA corresponds to protein coding mRNA, which do carry a poly(A) tail. However, in eukaryotic cells only about 1 to 5% of total RNA consists of primary transcripts, i.e. polyadenylated mRNA, whereas ribosomal RNA (rRNA) constitutes approximately 90% of the present RNA species. Thus, even when starting from poly(A) RNA, one major problem of transcriptome sequencing is the presence of interfering RNA molecules in particular if the poly(A) RNA starting material is not of sufficient purity and contains non-poly(A) contaminations. If abundant rRNA is involved in library construction, sequencing power will be used to sequence these ubiquitous molecules. The highly abundance of rRNA may predominate in the sequencing reads, thereby hindering the study of lowly expressed genes and wasting valuable sequencing resources. Furthermore, the presence of ribosomal RNA may result in a low signal-to-noise ratio that can make detection of the RNA species of interest difficult. Therefore, improving the depletion of rRNAs and/or other unwanted non-poly(A) RNA during isolation of the poly(A) RNA increases the value of the downstream sequencing because more information can be deduced from a sequencing run.

However, despite the fact that many methods are available in the art for isolating poly(A) nucleic acids, prior art methods have disadvantages. There is usually a trade-off between the poly(A) nucleic acid recovery and the achieved depletion of unwanted non-poly(A) nucleic acids. Methods which efficiently deplete unwanted non-poly(A) nucleic acids often suffer from poor poly(A) nucleic acid recovery rates. Other methods achieve good poly(A) nucleic acid recovery rates, but the depletion of non-poly(A) nucleic acids such as rRNA is insufficient. In fact, available methods usually require two or more rounds of poly(A) nucleic acid enrichment to provide poly(A) nucleic acid samples wherein the amount of non-poly(A) nucleic acid such as e.g. rRNA is sufficiently low to provide a useful sample for certain applications such as NGS applications where the purity requirements are high. This requirement for repetitions of the entire poly(A) nucleic acid isolation procedure can lead to several disadvantageous side effects and furthermore, extends the hours required to perform the procedure. The representational distribution of various poly(A) nucleic acids may become altered or more altered, or the unavoidable losses associated with the repeated procedure may reduce the level of the commonly-sought low-abundance messages beyond the limits of detection. These drawbacks pose major problems in particular in diagnostic settings and NGS applications where simplicity, efficient depletion of non-poly(A) nucleic acids, speed and reliability are driving characteristics for a viable poly(A) nucleic acid isolation method, in particular for a poly(A) RNA isolation method.

It is an object of the present invention to provide an improved method for isolating poly(A) nucleic acids such as in particular poly(A) RNA. In particular, it is an object to provide a method for isolating poly(A) nucleic acids, which efficiently enriches poly(A) nucleic acids while efficiently depleting non-poly(A) nucleic acids. Furthermore, one aim is to provide an improved method for isolating poly(A) nucleic acids for next generation sequencing. Furthermore, products suitable for performing respective methods shall be provided.

### SUMMARY OF THE INVENTION

The present invention is *inter alia* based on the surprising finding that nucleic acids containing a single stranded poly(A) stretch (in the following also referred to as "poly(A) nucleic acids") can be efficiently and specifically isolated using a capture probe and hybridization conditions which involve the use of a sodium salt in combination with a quaternary ammonium salt. Contaminations with non-poly(A) nucleic acids are reduced when using the hybridisation conditions described herein. As is demonstrated by the examples, the hybridization conditions used in the method according to the invention ensure efficient capture and hence isolation of the poly(A) nucleic acids while significantly reducing binding and hence carry-over of unwanted non-poly(A) nucleic acids into the isolated poly(A) nucleic acids. This results in a highly selective poly(A) nucleic acid recovery that can even be achieved in one isolation/enrichment step. This also increases the speed of the process compared to prior art methods which require two or more isolation cycles. The invention can be used to specifically and efficiently isolate poly(A) RNA from a nucleic acid containing sample while depleting unwanted non- poly(A) RNA such as rRNA during the isolation process. The method is particularly suitable for preparing poly(A) RNA for next generation sequencing (NGS) applications such as transcriptome sequencing. For such applications it is particularly important to efficiently capture the poly(A) RNA while removing non-poly(A) RNA, because the more non-interesting RNAs such as rRNAs are diminished in the isolated poly(A) RNA, the more information can be obtained from one sequencing run. Therefore, the method described herein makes an important contribution to the art.

Furthermore, it was found that the advantageous hybridization conditions which use a sodium salt in combination with a quaternary ammonium salt not only provide stringent and selective capture conditions for poly(A) nucleic acids, but also provide advantageous washing conditions. The hybridization conditions described herein therefore, can be advantageously used to remove non-poly(A) nucleic acids that may have bound to the capture probe and/or the captured poly(A) nucleic acids during washing.

Therefore, according to a first aspect, a method for isolating poly(A) nucleic acids having a single stranded poly(A) stretch from a nucleic acid containing sample is provided comprising:
(a) providing a hybridization composition comprising:
   i) a nucleic acid containing sample;
   ii) a hybridization solution comprising:
      aa. a sodium salt;
      bb. a quaternary ammonium salt;
         wherein the components of the hybridization solution can be added as single solution to the sample or may be added separately in any order to the sample;
   iii) a capture probe capable of hybridizing to the poly(A) stretch of the poly(A) nucleic acids;
      and incubating said hybridization composition under conditions to form nucleic acid-hybrids between the poly(A) nucleic acids and the capture probe;
(b) separating the formed hybrids from the remaining sample.

According to a second aspect, a method for sequencing poly(A) nucleic acids is provided comprising:
(a) isolating poly(A) nucleic acids from a nucleic acid containing sample using the method according to the first aspect;
(b) sequencing the isolated poly(A) nucleic acid molecules.

This method is particularly suitable for sequencing poly(A) RNA.

According to a third aspect, an aqueous hybridization solution suitable for hybridizing poly(A) nucleic acids to a capture probe capable of hybridizing to the poly(A) stretch of the poly(A) nucleic acids is provided, said hybridization solution comprising:
aa. a sodium salt in a concentration of ≤ 500 mM;
bb. a quaternary ammonium salt.

The hybridization solution according to the third aspect can used in conjunction with and for performing the methods according to the first, second and fourth aspect of the invention. It is particularly suitable for establishing the binding conditions for hybridizing poly(A) nucleic acids to a capture probe while preventing hybridization of non-poly(A) nucleic acids. Furthermore, it can be used to provide stringent washing conditions in order to remove bound non-poly(A) nucleic acids during the washing steps while maintaining hybridization of the poly(A) nucleic acids to the capture probe, thereby increasing the purity of the isolated poly(A) nucleic acid.

According to a fourth aspect, a kit is provided for isolating poly(A) nucleic acids from a nucleic acid containing sample, comprising:
(a) a hybridization solution according to the third aspect;
(b) a capture probe capable of hybridizing to the poly(A) stretch of the poly(A) nucleic acid.

According to a fifth aspect, a method is provided for isolating poly(A) nucleic acids having a single stranded poly(A) stretch from a nucleic acid containing sample comprising:
(a) hybridizing poly(A) nucleic acids to a capture probe capable of hybridizing to the poly(A) stretch of the poly(A) nucleic acids to form nucleic acid-hybrids between the poly(A) nucleic acid and the capture probe;
(b) separating the formed hybrids from the remaining sample;
(c) washing the separated hybrids with a hybridization solution according to the third aspect, wherein the components of the hybridization solution can be added as single solution to the hybrids or may be added separately in any order to the hybrids to generate the hybridization solution used for washing;
(d) releasing poly(A) nucleic acids from the washed hybrids.

Here, the advantageous hybridization solution comprising a sodium salt and a quaternary ammonium salt provided by the present invention is used in the washing step of the isolation process in order to reduce non-poly(A) nucleic acid contaminations during the washing step.

### DETAILED DESCRIPTION OF THE INVENTION

The present invention is *inter alia* based on the surprising finding that poly(A) nucleic acids can be efficiently isolated using a capture probe that can hybridize to the poly(A) stretch (e.g. poly(A) tail) of poly(A) nucleic acids and using hybridization conditions which involve the use of a sodium salt in combination with a quaternary ammonium salt. The hybridization conditions described herein ensure good poly(A) nucleic acid recovery while reducing the amount of unwanted non-poly(A) nucleic acids in the isolated poly(A) nucleic acids.

### METHOD FOR ISOLATING POLY(A) NUCLEIC ACIDS

In a first aspect a method is provided for isolating poly(A) nucleic acids having a single stranded poly(A) stretch from a nucleic acid containing sample comprising:
(a) providing a hybridization composition comprising:
   i) a nucleic acid containing sample;
   ii) a hybridization solution comprising:
      aa. a sodium salt;
      bb. a quaternary ammonium salt;
         wherein the components of the hybridization solution can be added as single solution to the sample or may be added separately in any order to the sample;
   iii) a capture probe capable of hybridizing to the poly(A) stretch of the poly(A) nucleic acids;
      and incubating said hybridization composition under conditions to form nucleic acid-hybrids between the poly(A) nucleic acids and the capture probe;
(b) separating the formed hybrids from the remaining sample.

The key advantages were described above. The individual method steps and preferred embodiments will be described subsequently.

### Step (a)

In step (a) a hybridization composition is provided comprising i) a nucleic acid containing sample from which the poly(A) nucleic acids shall be isolated, ii) a hybridization solution comprising a sodium salt and a quaternary ammonium salt and iii) a capture probe capable of hybridizing to the poly(A) stretch of the poly(A) nucleic acid. Said hybridization composition is incubated under conditions that allow the formation of nucleic acid - hybrids between the poly(A) nucleic acids and the capture probe.

The nucleic acid containing sample from which the poly(A) nucleic acids are isolated can be any sample from which poly(A) nucleic acids such as poly(A) RNA are commonly isolated. Details with respect to said nucleic acid containing sample and non-limiting common examples are also described below. According to one embodiment, poly(A) RNA is isolated from total RNA.

First, the hybridization conditions are explained subsequently, as they are decisive for achieving efficient binding of poly(A) nucleic acids to the capture probe while non-poly(A) nucleic acids cannot effectively bind and thus are efficiently depleted during the isolation process.

### The hybridization composition and the hybridization solution

The composition of the hybridization composition and hence the hybridization conditions will be explained subsequently. Providing the hybridization composition encompasses the addition of a hybridization solution to the sample. The components of the hybridization solution are preferably added as single solution to the sample. However, the components of the hybridization solution may also be added separately in any order to the sample e.g. using two or more solutions each containing at least one agent such as the sodium salt and/or the quaternary ammonium salt of the hybridization solution to generate the "hybridization solution" in the sense of the present disclosure. Such procedure also renders a "hybridization solution" and therefore, is encompassed by said term.

Subsequently, suitable and preferred embodiments of the hybridization composition and the hybridization solution suitable to establish these hybridization compositions are disclosed.

When describing and defining the concentration of components "of the hybridization solution" in the hybridization composition according to the present invention in the description and the claims of the present application, the volume contributed by the capture probe and the solid support (if a volume increasing solid support is used for assisting the capture and separation step) is not considered in the determination of the concentration of the subsequently described components in the hybridization composition. I.e. the capture probe and any solid support or other binding agent used to assist the separation (if used) are neglected in the calculation of the concentration of individual components in the hybridization composition. Furthermore, when describing and defining the concentration of components "of the hybridization solution" in the hybridization composition according to the present invention, corresponding components such as e.g. sodium chloride or a quaternary ammonium salt that are not added by the hybridization solution but are contained in the nucleic acid containing sample (if contained therein) are according to one embodiment not considered in the calculation of the concentration of the respective components. The concentrations and concentration ranges specified below were calculated neglecting the composition of the nucleic acid containing sample which is, as described herein, preferably a purified nucleic acid sample, that may also be diluted. However, according to another embodiment, respective components of the nucleic acid containing sample such as the sodium salt and/or the quaternary ammonium salt are considered in the calculation and in this embodiment, the salt concentration in the hybridization composition can be adjusted by the addition of the hybridization solution to yield the final concentration of the sodium salt and of the quaternary ammonium salt (provided by the nucleic acid containing sample and the hybridization solution) in the hybridization composition according to the specifications as given below.

According to one embodiment, the hybridization solution comprises its components in a concentrated form that allows it to be diluted with the nucleic acid containing sample and/or a dilution solution so as to achieve the proper final concentration in the hybridization composition when mixed with the nucleic acid containing sample and/or the dilution solution. Using a concentrated hybridization solution has the advantage that the user is more flexible with respect to the volume of the nucleic acid containing sample material and that the overall volume to be processed remains lower. E.g. the amount of nucleic acid containing sample can be adjusted to a certain volume by adding a dilution solution such as water or other appropriate solvent and said volume is then mixed with a predetermined volume of the hybridization solution to provide the hybridization conditions of the hybridization composition. This procedure has advantages, because the same volume of (diluted) sample material to a predetermined volume of the hybridization solution can be provided simply by adding the appropriate amount of a suitable dilution solution to the nucleic acid containing sample. E.g. 1 volume of (potentially diluted) nucleic acid containing sample can be contacted with 1 to 10, 1 to 5, 1 to 3 or 1 to 2 volumes of hybridization solution, depending on the composition of the hybridization solution. A ratio of 1 volume (potentially diluted) nucleic acid containing sample to 1 volume hybridization solution (1:2 dilution) is particularly preferred and was also used in several examples.

The hybridization solution and hence the hybridization composition comprises a sodium salt. This also encompasses the use of a mixture of different sodium salts as "a" sodium salt. The sodium salt promotes binding of the poly(A) nucleic acids to the capture probe. It may be an anorganic or organic sodium salt. According to one embodiment, the sodium salt is not a chaotropic salt and accordingly, the sodium salt is a non-chaotropic sodium salt. According to this embodiment, the hybridization solution or hybridization composition does not contain any chaotropic sodium salt. According to one embodiment, the sodium salt is a sodium halide. Preferably, the sodium halide is sodium chloride. It was found that sodium chloride is particularly suitable, because it renders advantageous hybridization conditions when used according to the teachings of the present invention.

During hybridization, the sodium salt must be present in a sufficient concentration to promote hybridization of the poly(A) nucleic acid to the capture probe. Hence, the hybridization composition comprises the sodium salt of the hybridization solution in a concentration wherein it is effective to promote hybridization of the poly(A) nucleic acids to the capture probe. However, with respect to the desired reduction of non-poly(A) nucleic acid contaminations, it was found advantageous to reduce the concentration of the sodium salt in the hybridization composition. Thus, according to one embodiment, the hybridization composition comprises the sodium salt of the hybridization solution in a concentration ≤ 250 mM. The hybridization composition may comprise the sodium salt of the hybridization solution in a concentration selected from 25 mM to 250 mM, 35 mM to 200 mM, 40 mM to 175 mM, 50 mM to 150 mM, 55 mM to 125 mM, 60 mM to 115 mM and 60 mM to 100 mM.

According to one embodiment, the hybridization solution used to establish the hybridization conditions in the hybridization composition comprises the sodium salt in a concentration ≤ 500 mM. The hybridization solution may comprise the sodium salt in a concentration that lies in a range selected from 50 mM to 500 mM, 75 mM to 400 mM, 85 mM to 350 mM, 100 mM to 300 mM, 115 mM to 250 mM, 120 mM to 225 mM and 125 mM to 200 mM. According to one embodiment, the hybridization solution comprises the sodium salt is a concentration that lies in a range selected from 125 mM to 175 mM. As is demonstrated by the examples, such hybridization solution provides particularly good results when being contacted with an equal volume of sample (or diluted sample). Thereby, hybridization conditions may be established in the hybridization composition as described in the previous paragraph. As mentioned, sodium chloride is preferably used as sodium salt.

According to a preferred embodiment, the hybridization composition comprises the sodium salt of the hybridization solution in a concentration that is ≤ 200 mM, ≤ 175 mM, ≤ 150 mM, ≤ 125 mM or ≤ 100 mM. Such lower concentrations of the sodium salt in the hybridization composition are preferred, as they provide stringent conditions for specific hybridizing the poly(A) nucleic acids to the capture probe, whereas unspecific hybridization of non-poly(A) nucleic acids is highly diminished. As is shown by the examples, a reduction of the sodium salt concentration in the hybridization solution/hybridization composition had a dramatic effect on non-poly(A) nucleic acid binding. Their binding was significantly reduced which resulted in less non-poly(A) contaminations in the isolated poly(A) nucleic acid. Said reduction in the salt concentration had, in relative terms, less influence on the hybridization of poly(A) nucleic acids to the capture probe. Thus, in relative terms, the effect of the reduced sodium salt concentration on the unwanted non-poly(A) nucleic acids was more severe than on the poly(A) nucleic acids. Still, reducing the salt concentration also reduced and hence negatively affected the poly(A) nucleic acid yield what is not desired.

It was surprisingly found though by the inventors that this disadvantageous effect on the poly(A) nucleic acid yield that is associated with a reduction of the sodium salt concentration in the hybridization composition is overcome when additionally including a quaternary ammonium salt in the hybridization solution and hence in the hybridization composition. Therefore, this combination maintains the advantageous effect regarding the depletion of non-poly(A) nucleic acids during poly(A) nucleic acid isolation while overcoming and thus compensating the reduction in poly(A) nucleic acid yield associated with the reduction of the sodium salt concentration. Using a sodium salt such as sodium chloride in combination with a quaternary ammonium salt therefore provides hybridization conditions that advantageously provide good poly(A) nucleic acid yields while effectively reducing contaminations of the isolated poly(A) nucleic acid with non-poly(A) nucleic acids.

Therefore, the hybridization solution and hence the hybridization composition comprises a quaternary ammonium salt. This also encompasses the use of a mixture of different quaternary ammonium salts as "a" quaternary ammonium salt. According to one embodiment, the quaternary ammonium salt is a tetraalkylammonium salt. The tetraalkylammonium salt may be a tetramethylammonium salt (TMA) or a tetraethylammonium salt (TEA). Suitable tetraalkylammonium salts include but are not limited to tetraethylammonium chloride (TEAC), tetramethylammonium chloride (TMAC), tetraethylammonium nitrate (TEAN), tetramethylammonium nitrate (TMAN), tetraethylammonium bromide (TEAB) and tetramethylammonium bromide (TMAB). According to one embodiment, the quaternary ammonium salt is not tetramethylammonium sulfate.

According to a preferred embodiment, the quaternary ammonium salt is a tetraalkylammonium salt selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB). Preferably, tetramethylammonium bromide is used as quaternary ammonium salt.

During hybridization, the quaternary ammonium salt is present in a concentration wherein it can support in combination with the sodium salt binding of the poly(A) nucleic acid to the capture probe. According to one embodiment, the hybridization composition comprises the quaternary ammonium salt of the hybridization solution in a concentration ≤ 3 M, ≤ 2.5 M, ≤ 2 M or ≤ 1.5 M. The hybridization composition may comprise the quaternary ammonium salt in a concentration ≥ 100 mM, ≥ 125 mM, ≥ 250 mM or ≥ 375mM. Preferably, the hybridization composition comprises the quaternary ammonium salt in a concentration selected from 0.1 M to 1.75 M, 0.125 M to 1.5 M, 0.25 M to 1.25 M, 0.375 M to 1 M and 0.375 M to 0.75 M. As described above, the quaternary ammonium salt preferably is a tetraalkylammonium salt and suitable examples were described above.

According to one embodiment, the hybridization solution used for establishing the hybridization conditions in the hybridization composition comprises the quaternary ammonium salt in a concentration ≤ 6 M, ≤ 5 M, ≤ 4 M or ≤ 3 M. The hybridization solution may comprise the quaternary ammonium salt in a concentration ≥ 200 mM, ≥ 250 mM, ≥ 500 mM or ≥ 750 mM. Preferably, the hybridization solution comprises the quaternary ammonium salt in a concentration selected from 0.2 M to 3.5 M, 0.25 M to 3M, 0.5 M to 2.5 M, 0.75 M to 2 M and 0.75 M to 1.5 M. As is demonstrated by the examples, such hybridization solution provides particularly good results when being contacted e.g. with an equal volume of sample (or diluted sample). Thereby, hybridization conditions may be established in the hybridization composition as described in the previous paragraph.

As is demonstrated by the examples, respective concentrations of the sodium salt, which preferably is sodium chloride and the quaternary ammonium salt, which preferably is a tetraalkylammonium salt such as tetramethylammonium bromide, in the hybridization composition and/or hybridization solution provide particularly good results. Suitable concentrations can also be determined by the skilled person following the teachings provided herein.

The hybridization solution and/or the hybridization composition may comprise further components, non-limiting embodiments are described subsequently.

According to one embodiment, the hybridization composition comprises a detergent. As is demonstrated by the examples, a detergent is not required when following the teachings of the present invention. However, it may be used e.g. to support the denaturation of secondary structures in the poly(A) nucleic acids thereby encouraging hybridization to the capture probe to the poly(A) tail. The detergent may be selected from ionic, zwitterionic and non-ionic detergents. Respective detergents and their use in hybridization reactions are well-known to the skilled person. According to one embodiment, an ionic detergent is used. Ionic detergents comprise anionic and cationic detergents. As is demonstrated by the examples, a hybridization composition comprising an anionic detergent such as SDS or LiDS provides good results. Suitable concentration ranges for the detergent in the hybridization composition include e.g. 0.025% to 5% and may be selected from 0.05% to 3%, 0.75% to 2.5%, 0.1% to 2.25% and 0.15% to 2%. The detergent is preferably comprised in and hence added by the hybridization solution. Suitable concentration ranges for the detergent in the hybridization solution include e.g. 0.05% to 10% and may be selected from 0.1% to 7.5%, 0.25% to 5%, 0.5% to 3% and 0.75% to 2%. Also lower concentration ranges of 0.1 % to 2%, 0.15% to 1% and 0.2% to 0.5% are suitable. The detergent could, however, also origin from a treatment of the sample depending on the sample type processed. E.g. if a lysate is processed as nucleic acid containing sample and a detergent was used to lyse the sample. However, a detergent is not required. Therefore, according to one embodiment, the hybridisation composition does not contain a detergent.

Furthermore, the hybridization composition may comprise a chelating agent. Chelating agents include, but are not limited to ethylenedinitrilotetraacetic acid (EDTA), diethylenetriaminepentaacetic acid (DTPA), ethylene glycol tetraacetic acid (EGTA) and N,N-bis(carboxymethyl)glycine (NTA) and furthermore, e.g. citrate or oxalate. According to a preferred embodiment, EDTA is used as chelating agent. As used herein, the term "EDTA" indicates *inter alia* the EDTA portion of an EDTA compound such as, for example, Na₂EDTA, K₂EDTA or K₃EDTA. Suitable concentration ranges for the chelating agent in the hybridization composition include e.g. 0 to 0.25M, 0.5mM to 50mM, 0.75mM to 10mM and 1mM to 5mM. The chelating agent is preferably comprised in and thus added by the hybridization solution. According to one embodiment, the hybridization solution comprises the chelating agent in a concentration that is selected from the range 0 to 0.5M, 1mM to 100mM, 1.5mM to 20mM and 2mM to 10mM. The chelating agent could, however, also origin from a treatment of the sample depending on the sample type processed.

According to one embodiment, the hybridization solution comprises a buffering agent such as e.g. Tris or other biological buffering agent such as MOPS, HEPES, MES or BIS-TRIS. Suitable examples of buffering agents are also known to the skilled person.

The pH value of the hybridization solution may e.g. lie in a range selected from 6 to 9. The hybridization solution may also comprise water or other solvent suitable to dissolve the components of the hybridization solution.

Furthermore, as described above, water or another suitable dilution solution can be added separately to the sample to dilute the sample and adjust a certain volume for the (diluted) nucleic acid containing sample that is then to be mixed with a certain volume of the hybridization solution. Such dilution also dilutes the hybridization solution, thereby assisting in establishing the hybridization conditions in the hybridization composition. As described herein, a respective dilution has the advantage that the hybridization solution can be provided in a concentrated form. Furthermore, variable sample volumes can be easily processed with the same amount of hybridization solution. For providing the hybridization composition, the given sample volume can be filled up with a dilution solution such as water to a given volume that is then mixed e.g. with an equal (or other predetermined) volume of hybridization solution. Also other ratios of sample/diluted sample to hybridization can be used depending on the composition of the hybridization solution.

According to one embodiment, the hybridization solution does not comprise chaotropic ions.

The hybridization conditions used in the method according to the invention are based on a balanced combination of a sodium salt and a quaternary ammonium salt which result in an efficient isolation of poly(A) nucleic acids while preventing carry-over of non-poly(A) nucleic acids. Therefore, the hybridization solution and respectively the hybridization composition does not contain other hybridization promoting salts besides the sodium salt and the quaternary ammonium salt in a concentration that would counteract these advantageous effects. Therefore, according to embodiments, the hybridization solution and/or hybridization composition does not contain hybridization promoting salts such as lithium or potassium chloride or other non-sodium halides, MgCl₂ and/or chaotropic salts in a concentration that would counteract the advantageous effects achieved by the combination of the sodium salt and the quaternary ammonium salt. In embodiments, the concentration of such salts if at all present, is 100mM or less, 75mM or less, 50mM or less or 25mM or less in the hybridization composition and/or hybridization solution. Preferably, the hybridization solution does not contain any hybridization promoting salts besides the sodium salt and the quaternary ammonium salt.

Non-limiting preferred embodiments of the hybridization composition and hybridization solution, in particular with respect to the contained sodium salt, which preferably is sodium chloride, and the quaternary ammonium salt, which preferably is a tetraalkylammonium salt, are described in the following. As discussed above, when describing the concentration of the components of the hybridization solution in the hybridization composition, the volume contributed by the capture probe and means used to assist the separation, in particular the solid support (if used for assisting the capturing and separation) is not considered in the determination of the concentration of the described components in the hybridization composition. Furthermore, as described, the components of the hybridization solution can be added as single solution to the sample or may be added separately in any order to the sample, e.g. using two or more solutions comprising at least one chemical of the hybridization solution to generate the hybridization solution.

According to one embodiment, the hybridization composition comprises the sodium salt of the hybridization solution in a concentration ≤ 250 mM and a tetraalkylammonium salt as quaternary ammonium salt. To ensure efficient binding, the concentration should be ≥ 25 mM, preferably ≥ 35 mM, more preferred ≥ 50mM. For providing a respective hybridization composition, a hybridization solution may be used which comprises a sodium salt in a concentration ≤ 500 mM and a tetraalkylammonium salt as quaternary ammonium salt. According to one embodiment, the hybridization solution comprises the sodium salt in a concentration of ≥ 50 mM, preferably ≥ 75 mM, more preferred ≥ 100 mM.

According to one embodiment, the hybridization composition comprises the sodium salt of the hybridization solution in a concentration selected from 25 mM to 175 mM, 50 mM to 150 mM, 55 mM to 125 mM, 60 mM to 115 mM and 60 mM to 100 mM and a tetraalkylammonium salt as quaternary ammonium salt in a concentration selected from 0.25M to 1.25M, 0.375 M to 1 M and 0.375 M to 0.75 M salt. For providing a respective hybridization composition, a hybridization solution may be used which comprises a sodium salt in a concentration selected from 50 mM to 350 mM, 100 mM to 300 mM, 115 mM to 250 mM, 120 mM to 225 mM and 125 mM to 200 mM and a tetraalkylammonium salt as quaternary ammonium salt in a concentration selected from from 0.5 M to 2.5 M, 0.75 M to 2 M and 0.75M to 1.5 M.

According to one embodiment, the hybridization composition comprises the sodium salt of the hybridization solution in a concentration selected from 37.5 mM to 125 mM, 50 mM to 100 mM and 55 mM to 87.5 mM and 60 mM to 100 mM and a tetraalkylammonium salt as quaternary ammonium salt in a concentration selected from 0.25M to 1.25M, 0.375 M to 1 M and 0.375 M to 0.75 M, wherein the sodium salt is sodium chloride. For providing a respective hybridization composition, a hybridization solution may be used which comprises sodium chloride in a concentration selected from 75 mM to 250 mM, 100 mM to 200 mM and 125 mM to 175 mM and a tetraalkylammonium salt selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB) as quaternary ammonium salt in a concentration selected from from 0.5 M to 2.5 M, 0.75 M to 2 M and 0.75 M to 1.5 M. As described, the use of tetraethylammonium bromide (TEAB) is particularly preferred.

As described above, a detergent may additionally be comprised in the hybridization composition and can be introduced by the hybridization solution. Details are described above and it is referred to the respective disclosure.

### Capture probe

The hybridization composition furthermore comprises a capture probe capable of hybridizing to the single-stranded poly(A) stretch of the poly(A) nucleic acids. The capture probe may be at least partially complementary to the single-stranded poly(A) stretch of the poly(A) nucleic acids, thereby allowing hybridization of the poly(A) nucleic acids via the poly(A) stretch such as in particular their poly(A) tail to the capture probe. Usually, the capture probe will comprise a single-stranded sequence that can hybridize to the poly(A) stretch of the poly(A) nucleic acid. It may also consist of such sequence. A hybridization composition comprising "a" capture probe as used herein also encompasses embodiments wherein the hybridization composition comprises two or more different capture probes.

Preferably, the capture probe is a capture oligonucleotide. The capture oligonucleotide sequence is designed to provide sufficient complementarity to the poly(A) stretch of poly(A) nucleic acids to allow specific hybridization to the poly(A) stretch that is sufficiently strong for the subsequent separation procedure, wherein the captured poly(A) nucleic acid is separated from non-poly(A) nucleic acids and other contaminations. Capture oligonucleotides that are suitable for such purpose are well-known in the art and therefore, do not need any detailed description. Nevertheless, some non-limiting embodiments are described subsequently.

The capture probe may comprise or may consist of RNA, DNA, PNA (peptide nucleic acid), LNA (locked nucleic acid) and/or other analogs. In particular analogs of the nucleobase T or U may be used insofar as they allow for hybridization with A residues. Any capture probe that is capable of hybridizing to the poly(A) stretch of poly(A) nucleic acids and hence is e.g. capable of sequence-specific binding to the poly(A) nucleic acid is considered "a capture probe". As mentioned, the capture probe is preferably a capture oligonucleotide such as a synthetic oligonucleotide. The capture oligonucleotide may comprise a poly-pyrimidine sequence capable of hybridizing to the poly(A) stretch such as the poly(A) tail of the poly(A) nucleic acid or a portion thereof, thereby allowing capture of the poly(A) nucleic acid upon hybridization. According to embodiments, the capture oligonucleotide comprises at least a stretch of contiguous units such as nucleobases or analogs thereof complementary to the poly(A) stretch, said stretch of the capture oligonucleotide preferably being at least 10, at least 12, at least 14, at least 16, at least 18, at least 20, at least 25, at least 30, at least 35, at least 40 units long as is well-known in the art. Also longer stretches of at least 50 or more units can be used in the capture oligonucleotide. Preferably, the capture oligonucleotide is at least in the region of complementarity to the poly(A) stretch single-stranded. Also the whole oligonucleotide may be single-stranded and/or complementary to the poly(A) stretch, respectively to the poly(A) tail. According to one embodiment, at least 60%, at least 70%, at least 80%, at least 90%, at least 95% or at least 100% of all pairing units (e.g. nucleobases) of the capture region and/or of the entire capture oligonucleotide are capable of hybridizing to at least a portion of the poly(A) stretch of poly(A) nucleic acids. According to one embodiment, when the capture oligonucleotide hybridizes to the poly(A) stretch of the poly(A) nucleic acid, a double-stranded nucleic acid hybrid is formed, which does not contain any mismatches.

According to one embodiment, the capture oligonucleotide is a poly(T) or poly(U) nucleic acid molecule. Preferably, a poly(dT) nucleic acid molecule is used. The term a poly(dT) nucleic acid in particular refers to a nucleic acid molecule comprising DNA, that has more than 90% T residues or comprising a DNA region of at least 10, at least 20, at least 25, at least 30 or at least 35 contiguous T residues. Respective capture oligonucleotides are well-known and commonly used for capturing poly(A) nucleic acids such as poly(A) RNA. According to one embodiment, a poly(dT) nucleic acid is used as capture oligonucleotide for isolating poly(A) RNA, such as in particular poly(A) mRNA, which is a synthetic molecule of which greater than 90% is the nucleobase T. As described, also analogs of the nucleobase T or U may be used insofar as they allow for hybridization with A residues. Such capture oligonucleotides are also commonly referred to as oligo-dT and are well-known in the art.

The capture probe may be present in the hybridization composition in free form. The capture probe with the bound poly(A) nucleic acids may then be immobilized to a solid phase during or after the hybridization reaction. The oligonucleotide may also be labeled with a compound that reacts with a second compound that in turn is immobilized to a solid support. Alternatively, the capture probe is provided in an immobilized form wherein the capture probe is attached to a solid support. Preferably, a solid support functionalized with the capture probe is used and hence is comprised in the hybridization composition. Immobilization to the solid support may be achieved using techniques that are well-known and standard in the art. In some embodiments, the oligonucleotide is attached to a solid support using a linker structure.

The solid support may be provided by various materials, including but not limited to reaction vessels, microtiter plates, particles, magnetic particles, cellulose, columns, plates, membranes, filter papers and dipsticks or any other solid support that can be used in separation technologies. Any support can be used as long as it allows separation of a liquid phase. Different solid supports were also used in known methods for isolating poly(A) nucleic acids. According to one embodiment, the solid support is provided by particles commonly also referred to as beads. The particles used may be made of glass, silica, polymers, polystyrene-latex polymers, cellulose and/or plastic. According to a preferred embodiment, the solid support is provided by a suspension of particles that are functionalized with the capture probe. The use of magnetic particles is preferred. When using magnetic particles as solid support, they may have superparamagnetic, paramagnetic, ferrimagnetic or ferromagnetic properties. Respective magnetic particles can be easily separated by the aid of a magnetic field, e.g. by using a permanent magnet and therefore have advantages with respect to the processing. They are compatible with established robotic systems capable of processing magnetic particles. Here, different robotic systems exist that can be used to process the magnetic particles to which the hybrids of the capture probe and the poly(A) nucleic acid are bound. According to one embodiment, magnetic particles are collected at the bottom or the side of a reaction vessel and the remaining liquid sample is removed from the reaction vessel, leaving behind the collected magnetic particles to which the hybrids are bound. Removal of the remaining sample can occur by decantation or aspiration. In an alternative system the magnet, which is usually covered by a cover or envelope, plunges into the reaction vessel to collect the magnetic particles. In a further alternative system, the sample comprising the magnetic particles can be aspirated into a pipette tip and the magnetic particles can be collected in the pipette tip by applying a magnet e.g. to the side of the pipette tip. The remaining sample can then be released from the pipette tip while the collected magnet particles which carry the bound hybrids remain due to the magnet in the pipette tip. The collected magnetic particles can then be processed further. Such systems are also well-known in the prior art and are also commercially available (e.g. BioRobot EZ1, QIAGEN). Also other processing systems are known and can be used.

Particles may also be separated by filtration, centrifugation or by using spin columns that can be e.g. loaded with a suspension of particles as is well-known to the skilled person. When the solid support is centrifuged it may be pelleted or passed through a centrfugible filter apparatus or column.

In some embodiments, the capture probe may be biotinylated or otherwise labeled so as to facilitate separation of the hybrids. This embodiment is e.g. suitable when using capture oligonucleotides. Biotin can be derivatized to probe nucleotides, for example using linkers, without impairing the ability of the capture oligonucleotide to hybridize to the poly(A) nucleic acid. Because biotin reacts with avidin/streptavidin, avidin or streptavidin may be employed in conjunction with a biotinylated capture oligonucleotide. The avidin or streptavidin may be linked to a solid support, such as particles or the surface of a vessel where it may bind the biotinylated capture oligonucleotide. The solid support may then be separated from the remainder of the sample e.g. by removing the solid support from the remaining sample or *vice versa* to isolate the biotinylated capture oligonucleotide, which itself is hybridized to the poly(A) nucleic acid. The capture probes can also be labelled for separation using a number of different modifications that are well known to those of skill in the art. Non-limiting alternatives include labelling the capture probe with an epitope tag and utilizing an antibody or a binding fragment thereof that recognizes that epitope for capture, for example, labelling the oligonucleotides with digoxigenin and using an anti-digoxigenin antibody for capture. Furthermore, haptens may be used for conjugation e.g. with nucleotides or oligonucleotides. Commonly used haptens for subsequent capture include biotin (biotin-11-dUTP), dinitrophenyl (dinitrophenyl-11-dUTP). These modifications include for example fluorescent modifications. Commercially available fluorescent nucleotide analogs that may be incorporated include but are not limited to Cy3™-dCTP, Cy3™-dUTP, Cy™5-dCTP, fluorescein-12-dUTP, AlexaFluor®594-5-dUTP, AlexaFluor®-546-14-dUTP and the like. Fluorescein labels may also be used as a separation moiety using commercially available anti-fluorescein antibodies. Also suitable is the labelling with radioisotopes, enzyme labels and chemiluminescent labels.

Furthermore, in case the capture probe itself is not linked to a solid support, hybrid binding agents immobilized to a solid support may be used to facilitate separation of the formed hybrids, such as e.g. anti-hybrid binding agents such as anti-DNA/RNA antibodies or binding fragments thereof. Such embodiments are e.g. suitable in case a RNA/DNA hybrid is formed upon hybridization of the capture probe to the poly(A) nucleic acid. A respective hybrid binding agent could likewise be immobilized to a solid support according to the principles described above.

Thus, many established systems are available that achieve that hybrids formed between the capture probe and the poly(A) nucleic acid are eventually immobilized onto a solid support which facilitates the separation of the hybrids. As described, the use of a solid support such as particles functionalized with the capture probe is preferred for the ease of handling.

### Incubation conditions

The hybridization composition is incubated under conditions and for a sufficient time to allow hybridization of the poly(A) nucleic acid to the capture probe. As explained, thereby, nucleic acid-hybrids between the poly(A) nucleic acid and the capture probe are formed. The hybridization composition may be gently rocked or otherwise agitated during incubation.

In embodiments, the hybridization composition is first heated at a temperature between about 60°C and about 90°C, preferably 65°C to 75°C, prior to incubation under hybridization conditions. A respective heating step assists in disrupting e.g. secondary structures of the poly(A) nucleic acid thereby supporting that the poly(A) stretch such as the poly(A) tail is accessible for hybridization to the capture probe. A respective heating step may be performed for less than 10 min, less than 7 min and preferably less than 5 min. In embodiments, for hybridization, the hybridization composition is incubated at a temperature of 50°C or below, 45°C or below, preferably 40°C or below, more preferred at room temperature, to allow hybridization of the poly(A) RNA to the capture probe. A respective incubation step may be performed for at least 4 min, preferably for a time period that lies in a range of 5 min to 30 min. It is an advantage of the present method that no long incubation times are required and that the hybridization step, including the denaturing step if performed, can be completed in less than 30 min and even less than 20min. However, also longer incubation times can be used if desired.

After completion of step (a), nucleic acid-hybrids are formed between the poly(A) nucleic acids and the capture probe.

### Step (b)

In step (b), the formed hybrids are separated from the remaining sample. Thereby, the poly(A) nucleic acid is isolated from the sample. The remaining sample is depleted of poly(A) nucleic acids which are bound to the capture probe.

As described above, separation of the hybrids is preferably assisted by the use of a solid support to which the capture probe and/or the formed hybrids are bound. The solid support with the bound hybrids can be easily separated from the remaining sample. As described, depending on the solid support used, the solid support may be removed from the remaining sample or the remaining sample may be recovered leaving behind the solid support with the bound hybrids. Suitable solid supports as well as suitable separation procedures which allow to separate the solid support from the remaining sample are well-known and are also described above in conjunction with the capture probe and it is referred to the respective disclosure which also applies here. Suitable separation procedures are also well-known and available to the skilled person.

### Optional wash step (c)

In optional step (c), the separated hybrids are washed one or more times. Even though this washing step (c) is optional, it is preferably performed in order to support removal of unbound components and impurities that could interfere with certain downstream applications of the isolated poly(A) nucleic acids.

Thus, according to a preferred embodiment, one or more washing steps are performed in step (c) in order to further purify the separated poly(A) nucleic acids. This can be conveniently performed e.g. while the hybrids are immobilized to a solid support which preferably is provided by particles. Common washing solutions may be used and suitable embodiments are known to the skilled person. A suitable washing solution removes impurities but substantially does not release the poly(A) nucleic acid from the hybrid to present losses of poly(A) nucleic acids during washing.

As one or more washing solution, washing solutions having e.g. the same or a lower concentration of the sodium salt and/or having the same or a lower concentration of the quaternary ammonium salt compared to the used hybridization solution may be used. If more than one washing buffer is used, the salt concentration may be decreased between the washing steps.

When particles are used as solid support for immobilizing the capture probe, the particles may be resuspended by agitation, e.g. vortexing during washing.

### Optional release step (d)

In optional release step (d), the poly(A) nucleic acids are released from the hybrids. This can be conveniently performed e.g. while the hybrids are immobilized to a solid support which preferably is provided by particles. To achieve release, one or more elution steps may be performed in order to elute the captured poly(A) nucleic acids.

Here, basically any release solution can be used which effects release of the poly(A) nucleic acids from the hybrids and hence allows e.g. to elute the bound poly(A) nucleic acid from the solid support, such solid support is preferably used to assist the separation of the poly(A) nucleic acids. Respective elution solutions that effectively elute poly(A) nucleic acids from complementary capture probes are known to the skilled person and therefore need no detailed description. Non-limiting examples include water, elution buffers such as TE-buffer and low-salt solutions which have a salt content of 150mM or less, 100mM or less, 75mM or less, 50mM or less, 25mM or less, 20mM or less, 15mM or less, 10mM or less or are salt-free. The elution solution may e.g. comprise a buffering agent, in particular may comprise a biological buffer such as Tris, MOPS, HEPES, MES, BIS-TRIS, propane and others.

Elution may be assisted by heating, e.g. to 50°C or above, preferably 60°C or above, more preferred 65°C or above.

Elution may also be assisted by shaking what is e.g. particularly feasible if a particulate solid support is used.

Furthermore, it is also within the scope of the present invention to repeat the elution step in order to ensure that the captured poly(A) nucleic acid is efficiently released.

### Particular embodiments

Particularly preferred embodiments of the method according to the first aspect are described again in the following:
According to one embodiment, the method comprises:
   (a) providing a hybridization composition comprising:
      i) a nucleic acid containing sample;
      ii) a hybridization solution comprising:
         aa. a sodium salt in a concentration ≤ 500 mM;
         bb. a tetraalkylammonium salt as quaternary ammonium salt; wherein the components of the hybridization solution can be added as single solution to the sample or may be added separately in any order to the sample;
      iii) a capture probe capable of hybridizing to the poly(A) stretch of the poly(A) nucleic acids;
         wherein the hybridization composition comprises the sodium salt of the hybridization solution in a concentration ≤ 250 mM,
         and incubating said hybridization composition under conditions to form nucleic acid-hybrids between the poly(A) nucleic acids and the capture probe;
   (b) separating the formed hybrids from the remaining sample;
      and preferably, additionally comprises
   (c) washing the separated hybrids and
   (d) releasing poly(A) nucleic acids from the hybrids.

According to one embodiment, the method comprises:
(a) providing a hybridization composition comprising:
   i) a nucleic acid containing sample;
   ii) a hybridization solution comprising:
      aa. a sodium salt in a concentration selected from 50 mM to 350 mM, 75 mM to 300 mM, 100 mM to 250 mM and 125 mM to 200 mM;
      bb. a tetraalkylammonium salt as quaternary ammonium salt in a concentration selected from from 0.2 M to 2.5 M, 0.5 M to 2 M and 0.75 M to 1.5 M;
         wherein the components of the hybridization solution can be added as single solution to the sample or may be added separately in any order to the sample;
   iii) a capture probe capable of hybridizing to the poly(A) stretch of the poly(A) nucleic acids;
      wherein the hybridization composition comprises the sodium salt of the hybridization solution in a concentration selected from 25 mM to 175 mM, 37.5 mM to 150 mM, 50 mM to 125 mM and 62.5 mM to 100 mM and the tetraalkylammonium salt in a concentration selected from 0.1 M to 1.25M, 0.25 M to 1 M and 0.375 M to 0.75 M,
   and incubating said hybridization composition under conditions to form nucleic acid-hybrids between the poly(A) nucleic acids and the capture probe;
(b) separating the formed hybrids from the remaining sample;
   and preferably, additionally comprises
(c) washing the separated hybrids and
(d) releasing poly(A) nucleic acids from the hybrids.

According to one embodiment, the method is for isolating poly(A) RNA from a total RNA sample, comprising:
(a) providing a hybridization composition comprising:
   i) a nucleic acid containing sample;
   ii) a hybridization solution comprising:
      aa. a sodium salt which is sodium chloride in a concentration selected from 75 mM to 250 mM, 100 mM to 200 mM and 125mM to 175mM;
      bb. a tetraalkylammonium salt selected from the group consisting of
         tetraethylammonium chloride (TEAC), tetramethylammonium chloride (TMAC), tetramethylammonium nitrate (TMAN), tetraethylammonium bromide (TEAB) and tetramethylammonium bromide (TMAB) as quaternary ammonium salt in a concentration selected from from 0.25 M to 2.5 M, 0.5 M to 2 M and 0.75 M to 1.5 M; wherein the components of the hybridization solution can be added as single solution to the sample or may be added separately in any order to the sample;
   iii) a capture probe capable of hybridizing to the poly(A) stretch of the poly(A) nucleic acids wherein the capture probe is immobilized to a solid support, which preferably is provided by particles;
      wherein the hybridization composition comprises the sodium salt of the hybridization solution in a concentration selected from 37.5 mM to 125 mM, 50 mM to 100 mM and 62.5 mM to 87.5 mM and 62.5 mM to 100 mM and the tetraalkylammonium salt in a concentration selected from 0.125 M to 1.25 M, 0.5 M to 1 M and 0.375 M to 0.75 M,
      and incubating said hybridization composition under conditions to form nucleic acid-hybrids between the poly(A) nucleic acids and the capture probe;
(b) separating the formed hybrids bound to the solid support from the remaining sample;
   and preferably, additionally comprises
(c) washing the separated hybrids and
(d) releasing poly(A) nucleic acids from the hybrids.

### Application and uses of the method according to the first aspect

The method according to the first aspect allows to efficiently isolate poly(A) nucleic acids from various samples while minimizing a carry-over of non-poly(A) nucleic acids into the isolated poly(A) nucleic acids.

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. The term encompasses naturally occurring nucleic acids as well as synthetic nucleic acids.

As mentioned above, the term "poly(A) nucleic acid" and corresponding terms in particular refer to a nucleic acid comprising a single stranded poly(A) stretch of consecutive adenine base ("A") residues. Such poly(A) stretch is usually provided at the 3' end of a nucleic acid and then is also referred to as poly(A) tail. The term "poly(A) nucleic acid" specifically encompasses any nucleic acid species which comprises a poly(A) tail at its 3' end, in particular sequences having a poly(A) tail of at least 10, at least 15 or at least 20 A residues. The term poly(A) nucleic acid refers to poly(A) RNA as well as poly(A) DNA. As described herein, the poly(A) nucleic acid preferably is poly(A) RNA, in particular poly(A) mRNA. Poly(A) RNA is the major polyadenylated nucleic acid of interest for research and diagnostic applications.

Non-poly(A) nucleic acids are substantially not captured with the method of the invention due to the advantageous hybridization conditions used. Non-poly(A) nucleic acids, i.e. nucleic acids not having a single-stranded poly(A) stretch such as a poly(A) tail, which are not captured and hence are efficiently depleted during the isolation process of the present invention comprise e.g. non-polyadenylated RNA such as rRNA, tRNA, snRNA, snoRNA and also the minor portion of mRNA lacking a poly(A). The term non-poly(A) nucleic acid may also refer to other non-polyadenylated nucleic acids such as non-polyadenylated DNA if present in the sample.

The term "nucleic acid containing sample" is used herein in a broad sense and is intended to include a variety of sources and compositions that contain nucleic acids. The nucleic acid containing sample may derive from a biological sample but the term also includes other, e.g. artificial samples which comprise poly(A) nucleic acids such as e.g. nucleic acids that were provided *in vitro* with a poly(A) tail. It in particular refers to compositions comprising purified nucleic acids that were isolated from a biological sample such as total RNA from which the poly(A) nucleic acid is to be isolated using the method of the invention. However, the nucleic acid containing sample may also be provided by a biological sample, in particular a lysate of a biological sample. As is known to the skilled person, poly(A) nucleic acids such as poly(A) RNA can be isolated from a sample lysate.

Exemplary biological samples include, but are not limited to, cell samples, environmental samples, samples obtained from a body, in particular body fluid samples, and human, animal or plant tissue samples. Non-limiting examples include, but are not limited to cells, whole blood, blood products, red blood cells, white blood cells, buffy coat, plasma, serum, swabs, urine, sputum, saliva, semen, lymphatic fluid, amniotic fluid, cerebrospinal fluid, peritoneal effusions, pleural effusions, biopsy samples, fluid from cysts, synovial fluid, vitreous humor, aqueous humor, bursa fluid, eye washes, eye aspirates, plasma, serum, pulmonary lavage, lung aspirates, animal, in particular human, or plant tissues, including but not limited to, liver, spleen, kidney, lung, intestine, brain, heart, muscle, pancreas, cell cultures, as well as lysates, extracts, or materials and fractions obtained from the samples described above. Preferably, the sample is a biological sample derived from a human, animal or plant. The sample may be selected from the group consisting of cells, tissue, tumor cells, and body fluids such as for example blood, blood products such as buffy coat, plasma and serum, urine, liquor, sputum, stool, CSF and sperm, epithelial swabs, biopsies, bone marrow samples and tissue samples, preferably organ tissue samples. The term "sample" also includes processed samples such as preserved, fixed and/or stabilised samples. As described above, the nucleic acid containing sample comprised in the hybridization composition is preferably provided by nucleic acids purified from a respective sample such as e.g. total RNA. However, the nucleic acid containing sample may also be a crude sample comprising the poly(A) nucleic acids in released form and may be provided by a lysate obtained from a respective biological sample.

According to a preferred embodiment, the nucleic acid containing sample is a purified nucleic acid sample. Preferably, the nucleic acid containing sample is total RNA and poly(A) RNA is isolated from said total RNA using the method of the invention. Total RNA can be isolated from various samples e.g. using any common RNA purification method. Suitable methods are well-known in the prior art and thus, do not need a detailed description here. Suitable methods include but are not limited to the isolation of RNA using phenol/chloroform based methods, the isolation of RNA using chaotropic agents, alcohol and a solid phase such as in particular a silicon containing solid phase (e.g. silica, glass fibers, silicon carbide), alcohol precipitation, precipitation by other organic solvents, polymers or cationic detergents and the like.

According to one embodiment, the nucleic acid containing sample from which the poly(A) nucleic acid, preferably poly(A) RNA, is isolated, is a processed biological sample such as a lysed sample. The sample may be lysed using any suitable lysis method that is compatible with poly(A) nucleic acid isolation. Various methods for lysing a biological sample are known to the skilled person and also depend on the specific sample type to be processed. The lysis may e.g. be based on or include a chemical or mechanical lysis procedure and non-limiting examples include lysis methods involving lytic agents such as e.g. detergents, proteolytic enzymes or chaotropic salts, heating, ultrasonification, mechanical disruption and the like. According to one embodiment, a DNA depleted lysate is used as nucleic acid containing sample. DNA may be removed from the lysate e.g. by performing a DNase digest or by selectively isolating and thus removing DNA from the lysate. Suitable methods for selectively binding and thus removing DNA are for example described in EP 0 880 537 and WO 95/21849, herein incorporated by reference. E.g. if lysing the sample using chaotropic agents such as chaotropic salts in the absence of short chained alcohols such as ethanol or isopropanol, binding conditions can be established that are selective for DNA, in particular if a silicon containing solid phase is used. If desired, the bound DNA can be further used, e.g. further processed, e.g. sequenced, and thus may e.g. optionally be washed and eluted from the nucleic acid binding solid phase thereby providing a DNA fraction which is substantially free of RNA. However, if the DNA is not of interest, the bound DNA may also be simply discarded if only RNA is of interest. Furthermore, the RNA containing lysate may be cleared prior to isolating the poly(A) RNA therefrom in order to remove e.g. cell debris and other contaminants.

The method according to the present invention is particularly suitable for isolating poly(A) RNA from eukaryotic samples. The method efficiently depletes abundant rRNA such as 28S rRNA, 18S rRNA, 5.8S rRNA, 5S rRNA, mitochondrial 12S rRNA and mitochondrial 16S rRNA. As described, in particular for next generation sequencing applications an efficient depletion of such unwanted RNA is mandatory as otherwise valuable sequencing capacity is wasted.

In experiments which used the method according to the present invention, non-poly(A) RNA such as 5S rRNA, 5.8S rRNA and 28S rRNA was depleted by more than 99%. Thus, according to one embodiment, the amount of non-poly(A) RNA is ≤ 3%, ≤ 2%, ≤ 1.5%, preferably ≤ 1%, ≤ 0.75%, ≤ 0.5%, ≤ 0.25%, ≤ 0.15%, ≤ 0.1%, ≤ 0.05% in the isolated poly(A) RNA. As mentioned, the non-poly(A) RNA to be depleted is in particular rRNA. Typical rRNA that needs to be efficiently depleted is 5S rRNA, 5.8S rRNA, 18S rRNA and 28S rRNA. Furthermore, also 12 mt and 16 mt rRNA can be efficiently depleted with the method of the invention.

The method can be used for the preparation of poly(A) nucleic acids for any purpose for which the isolation of poly(A) nucleic acids such as in particular poly(A) RNA is commonly desired. Non-limiting examples include, but are not limited to the isolation of poly(A) RNA from either total RNA or directly from lysates of biological samples such as cells and tissues, for cDNA synthesis, cDNA library construction, amplification based methods such as reverse transcription PCR, subtractive hybridization, the direct isolation of polyadenylated *in-vitro* transcripts, oligonucleotides or other nucleic acids, *in vitro* translation, SAGE technology, expression analysis, expression array and expression-chip analysis, microarray analysis, RNAse and S1 nuclease protection, primer extension, RNA northern, dot, and slot blotting, micro injection and furthermore, for sequencing applications, such as in particular NGS applications. It may also be used in order to selectively remove poly(A) nucleic acids from samples in case the poly(A) nucleic acids are undesired and shall be removed from a sample. In this case, e.g. washing and elution steps to further purify the isolated poly(A) nucleic acid may be obsolete. However, it may also be of interest to analyze the isolated and removed poly(A) nucleic acids separately from non-poly(A) nucleic acids and hence also wash and elute them in this case.

The method according to the invention is particularly advantageous because it is highly efficient thereby minimizing losses of poly(A) nucleic acids due to the isolation process, is highly selective for poly(A) nucleic acids thereby reducing unwanted carry-over of non-poly(A) nucleic acids into the isolated poly(A) nucleic acid fraction, and furthermore, requires minimal hands-on time. The advantageous results can be achieved already after one isolation cycle which is a considerable advantage over prior art methods, which often need at least two enrichment cycles in order to provide sufficiently pure poly(A) nucleic acids. Therefore, the method is highly suitable for simultaneous handling of multiple samples also in high throughput applications.

As mentioned above, the method according to the present invention is particularly suitable for preparing poly(A) RNA for next generation sequencing (NGS) applications such as transcriptome sequencing. For such applications it is particularly important to efficiently capture the poly(A) RNA while removing non-poly(A) RNA such as the abundant rRNA from the poly(A) RNA of interest during the isolation process because the more non-interesting RNAs such as rRNAs are diminished, the more information can be obtained from one sequencing run. The present invention provides such a method and therefore, makes an important contribution to the art. For this purpose, purified total RNA is preferably used as nucleic acid containing sample material. The poly(A) RNA enriched eluate that is obtained after the isolation process can be used for construction of a sequencing library. This embodiment will also be explained in further detail in conjunction with the method according to the second aspect and it is referred to the respective disclosure.

### METHOD FOR SEQUENCING POLY(A) NUCLEIC ACIDS

According to a second aspect, a method for sequencing poly(A) nucleic acids, preferably poly(A) RNA, is provided comprising:
(a) isolating poly(A) nucleic acids from a nucleic acid containing sample using the method according to the first aspect;
(b) sequencing the isolated poly(A) nucleic acid molecules.

In step (a), the method according to the first aspect is performed in order to isolate poly(A) nucleic acids from a sample. Details of said method and associated advantages are described above and we refer to the respective disclosure which also applies here. Preferably, poly(A) RNA is isolated as poly(A) nucleic acid. The poly(A) RNA is preferably isolated from a total RNA sample according to step (a) and (b) of the method according to the first aspect and is washed and eluted according to steps (c) and (d) of the method according to the first aspect as is described above.

The purified poly(A) nucleic acid is then sequenced in step (b). According to one embodiment, sequencing comprises preparing from the isolated poly(A) nucleic acids a sequencing library. Preferably, said sequencing library is suitable for massive parallel sequencing and sequencing comprises sequencing in parallel the molecules comprised in the library. Respective sequencing libraries are known in the art. A sequencing library may comprise a plurality of double-stranded molecules and preferably is suitable for massive parallel sequencing and accordingly, is suitable for next generation sequencing. Preparation of a respective sequencing library is also the present standard in transcriptome sequencing. The plurality of double stranded nucleic acid molecules present in the sequencing library may be linear or circular, preferably, the nucleic acid molecules comprised in the sequencing library are linear. A sequencing library which is suitable for next generation sequencing can be prepared using methods known in the prior art. Preferably, the double-stranded molecules in the sequencing library are DNA molecules. For this purpose, poly(A) RNA may be reverse transcribed to cDNA in case the poly(A) nucleic acid is poly(A) RNA. Usually, methods for preparing a sequencing library suitable for next generation sequencing include obtaining DNA fragments optionally followed by DNA repair and end polishing and, finally, often NGS platform-specific adaptor ligation. According to one embodiment, the obtained cDNA can be fragmented for example by shearing, such as sonification, hydro-shearing, ultrasound, nebulization or enzymatic fragmentation, in order to provide DNA fragments that are suitable for subsequent sequencing. However, fragmentation to the desired length may occur on the RNA level and thus prior to cDNA synthesis. E.g. the isolated poly(A) RNA may be fragmented by magnesium-catalysed hydrolysis of the RNA. The length of the fragments can be chosen based on the sequencing capacity of the next generation sequencing platform that is subsequently used for sequencing. Usually, the obtained fragments have a length of 1500bp or less, 1000bp or less, 750bp or less, 600bp or less and preferably 500bp or less as this corresponds to the sequencing capacity of most current next generation sequencing platforms. The fragmented DNA can be repaired afterwards and end polished using methods known in the prior art, thereby providing for example blunt ends or nucleotide overhangs, such as A overhangs.

Furthermore, adapters can be ligated at the 5' and/or 3' ends of the DNA fragments, preferably at both ends of the obtained fragments. The specific design of the adapters depends on the next generation sequencing platform to be used and for the purposes of the present invention, basically any adaptors used for preparing sequencing libraries for next generation sequencing can be used. Thus, the sequencing library may comprise or consist of randomly fragmented double stranded DNA molecules which are ligated at their 3' and 5' end to adapter sequences. The adaptors provide a known sequence and thus provide a known template for amplification and/or sequencing primers. As adaptors, double-stranded or partially double-stranded nucleic acids of known sequence can be used. The adapters may have blunt ends, cohesive ends with 3' or 5'overhangs, may be provided by Y shaped adapters or by stem-loop shaped adapters (see e.g. US 2009/0298075). Y shaped adapters are e.g. described in (see e.g. US 7,741,463). Optionally, the adapters may also provide an individual index thereby allowing the subsequent pooling of two or more sequencing libraries prior to sequencing. As discussed, sequencing is preferably performed on a next generation sequencing platform. In NGS, sequencing is often performed by repeated cycles of polymerase-mediated nucleotide extensions or, in one common format, by iterative cycles of oligonucleotide ligation. After obtaining the sequencing library using the method according to the present invention, clonal separation of single molecules and subsequent amplification is performed by in vitro template preparation reactions like emulsion PCR (pyrosequencing from Roche 454, semiconductor sequencing from Ion Torrent, SOLiD sequencing by ligation from Life Technologies, sequencing by synthesis from Intelligent Biosystems), bridge amplification on the flow cell (e.g. Solexa/Illumina), isothermal amplification by Wildfire technology (Life Technologies) or rolonies/nanoballs generated by rolling circle amplification (Complete Genomics, Intelligent Biosystems, Polonator). Sequencing technologies like Heliscope (Helicos), SMRT technology (Pacific Biosciences) or nanopore sequencing (Oxford Nanopore) allow direct sequencing of single molecules without prior clonal amplification. The sequencing can be performed on any of the respective platforms using a sequencing library prepared from the isolated poly(A) RNA. Suitable methods for preparing sequencing libraries and next generation sequencing methods are also described in Metzker, 2011, Voelkerding, 2009, and WO12/003374.

The advantages of preparing the poly(A) nucleic acid such as preferably poly(A) RNA in step (a) using the method of the first aspect with respect to the sequencing results are described above and it is referred to the respective disclosure.

### HYBRIDIZATION SOLUTION AND KIT

According to a third aspect, an aqueous hybridization solution is provided, comprising:
aa. a sodium salt in a concentration ≤ 500 mM;
bb. a quaternary ammonium salt.

The hybridization solution according to the third aspect can be used in conjunction with and for performing the method according to the first, second and fourth aspect of the invention and in particular it can be used to establish favourable binding conditions for capturing the poly(A) nucleic acid or it may can be used to provide stringent washing conditions that assist in removing non-poly(A) nucleic acids that may have been bound during the capture step, e.g. if the advantageous hybridization conditions according to the invention wherein a reduced concentration of a sodium salt is used in combination with a quaternary ammonium salt such as preferably a tetraalkylammonium salt are not used.

Details regarding the hybridization solution, in particular suitable and preferred hybridization solution components and hybridization solution component concentrations as well as suitable and preferred mixing ratios with the sample (or diluted sample) are described in detail above in conjunction with the method according to the first aspect of the present invention. It is referred to the above disclosure which also applies here. Non-limiting selected embodiments are again described briefly subsequently.

The hybridization solution may comprise the sodium salt in a concentration ≤ 500 mM. The sodium salt preferably is a sodium halide, more preferably sodium chloride. The hybridization solution may comprise the sodium salt in a concentration that lies in a range selected from 50 mM to 500 mM, 75 mM to 400 mM, 85 mM to 350 mM, 100 mM to 300 mM, 115 mM to 250 mM, 120 mM to 225 mM and 125 mM to 200 mM. According to one embodiment, the hybridization solution comprises the sodium salt in a concentration that lies in a range selected from 125 mM to 175 mM. As is demonstrated by the examples, such hybridization solution provides particularly good results when being contacted with an equal volume of sample (or diluted sample).

The hybridization solution may comprise the quaternary ammonium salt in a concentration ≤ 6 M, ≤ 5 M, ≤ 4 M or ≤ 3 M. The hybridization solution may comprise the quaternary ammonium salt in a concentration ≥ 200 mM, ≥ 250 mM, ≥ 500 mM or ≥ 750 mM. Preferably, the hybridization solution comprises the quaternary ammonium salt in a concentration selected from 0.2 M to 3.5 M, 0.25 M to 3M, 0.5 M to 2.5 M, 0.75 M to 2 M and 0.75 M to 1.5 M. The quaternary ammonium salt preferably is a tetraalkylammonium salt such as a tetramethylammonium salt (TMA) or a tetraethylammonium salt (TEA). Suitable tetraalkylammonium salts include but are not limited to tetraethylammonium chloride (TEAC), tetramethylammonium chloride (TMAC), tetraethylammonium nitrate (TEAN), tetramethylammonium nitrate (TMAN), tetraethylammonium bromide (TEAB) and tetramethylammonium bromide (TMAB). According to one embodiment, the quaternary ammonium salt is not tetramethylammonium sulfate. Preferably, the hybridization solution comprises tetramethylammonium bromide as quaternary ammonium salt.

According to one embodiment, the hybridization solution comprises one or more compounds selected from the group consisting of detergents, chelating agents and buffers. Details are described above in conjunction with the first aspect and it is referred to the above disclosure. According to one embodiment, the hybridization solution does not comprise chaotropic ions.

As described above, the hybridization conditions used in the method according to the invention are based on a balanced combination of a sodium salt and a quaternary ammonium salt which result in an efficient isolation of poly(A) nucleic acids while preventing carry-over on non-poly(A) nucleic acids. Therefore, the hybridization solution does not contain other hybridization promoting salts besides the sodium salt and the quaternary ammonium salt in a concentration that would counteract these advantageous effects. Therefore, according to embodiments, the hybridization solution does not contain hybridization promoting salts such as lithium or potassium chloride or other non-sodium halides, MgCl₂ and/or chaotropic salts in a concentration that would counteract the advantageous effects achieved by the combination of the sodium salt and the quaternary ammonium salt. In embodiments, the concentration of such salts if at all present in the hybridization solution, is 100mM or less, 75mM or less, 50mM or less or 25mM or less. Preferably, the hybridization solution does not contain any hybridization promoting salts besides the sodium salt and the quaternary ammonium salt.

According to one embodiment, the hybridization solution comprises a sodium salt in a concentration ≤ 500 mM and a tetraalkylammonium salt as quaternary ammonium salt. The hybridization solution may comprise the sodium salt in a concentration of ≥ 50 mM, ≥ 75 mM or ≥ 100 mM. The hybridization solution may comprise e.g. a sodium salt in a concentration selected from 50 mM to 350 mM, 100 mM to 300 mM, 115 mM to 250 mM, 120 mM to 225 mM and 125 mM to 200 mM and a tetraalkylammonium salt as quaternary ammonium salt in a concentration selected from from 0.5 M to 2.5 M, 0.75 M to 2 M and 0.75M to 1.5 M. E.g. the hybridization solution may comprise sodium chloride in a concentration selected from 75 mM to 250 mM, 100 mM to 200 mM and 125 mM to 175 mM and a tetraalkylammonium salt selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB) as quaternary ammonium salt in a concentration selected from from 0.5 M to 2.5 M, 0.75 M to 2 M and 0.75 M to 1.5 M. As described, the use of tetraethylammonium bromide (TEAB) is particularly preferred.

The hybridization solution according to the third aspect can be advantageously used for preparing a hybridization composition as explained above in conjunction with the method according to the first aspect. It is referred to the above disclosure which also applies here. Furthermore, it can be used to establish stringent washing conditions as will be described in detail below.

According to a fourth aspect, a kit is provided for isolating poly(A) nucleic acids from a sample, comprising:
(a) a hybridization solution according to the third aspect;
(b) a capture probe capable of hybridizing to the poly(A) stretch of the poly(A) nucleic acid.

The hybridization solution according to the third aspect is described above and it is referred to the above disclosure. Details and preferred embodiments with respect to the capture probe are also described in conjunction with the method according to the first aspect and it is referred to the above disclosure which also applies here. As described above, the capture probe may be a capture oligonucleotide such as preferably a synthetic oligo(T) - or oligo(U)-comprising nucleic acid molecule or a mixture thereof or any other suitable capture probe that is or can be immobilized to a solid support. Suitable and preferred embodiments of the solid support are also described in conjunction with the method according to the first aspect and it is referred to the above disclosure which also applies here. As described above, according to one embodiment the capture probe is bound to non-magnetic or magnetic particles.

The kit is particularly suitable for use in the method according to the first aspect. Furthermore, the kit may comprise instructions and/or information for use. E.g. the kit may comprise instructions and/or information regarding the application of a certain volume of the hybridization solution with a certain volume of the nucleic acid containing sample and/or a dilution solution such as water, to achieve effective concentrations of the sodium salt and the quaternary ammonium salt in the hybridization composition. Depending on the used volumes/ratios, different salt concentrations can be used in the hybridization solution.

### METHOD FOR WASHING POLY(A) NUCLEIC ACID CONTAINING HYBRIDS

It was also found by the inventors that the advantageous hybridization conditions which employ a reduced concentration of a sodium salt in combination with a quaternary ammonium salt not only provide highly stringent and selective capture conditions for poly(A) nucleic acids, but also provide highly stringent washing conditions. These hybridization conditions therefore, can be advantageously used to remove non-poly(A) nucleic acids during washing steps.

Hence, according to a fifth aspect, a method for isolating poly(A) nucleic acids having a single stranded poly(A) stretch from a nucleic acid containing sample is provided comprising:
(a) hybridizing poly(A) nucleic acids to a capture probe capable of hybridizing to the poly(A) stretch of the poly(A) nucleic acids to form nucleic acid-hybrids between the poly(A) nucleic acids and the capture probe;
(b) separating the formed hybrids from the remaining sample;
(c) washing the separated hybrids with a hybridization solution according to the third aspect, wherein the components of the hybridization solution can be added as single solution to the hybrids or may be added separately in any order to the hybrids to generate the hybridization solution used for washing;
(d) releasing poly(A) nucleic acids from the washed hybrids.

In step (a), poly(A) nucleic acids are hybridized to a capture probe capable of hybridizing to the poly(A) stretch of the poly(A) nucleic acids to form nucleic acid-hybrids. As described above, a capture oligonucleotide comprising a sequence complementary to the poly(A) tail may be used. In the method according to the fifth aspect, any method and hence hybridization conditions can be used for providing the respective hybrids. Therefore, also prior art methods can be used. However, it is preferred that the hybridization conditions described above in conjunction with the method according to the first aspect are also used in step (a) of the method according to the fifth aspect. Details with respect to the poly(A) nucleic acids and the capture probe are described in conjunction with the first aspect according to the present invention. The respective disclosure also applies here.

In step (b), the formed hybrids are separated from the remaining sample. Here, any common separation technology can be used. Exemplary embodiments are described above in conjunction with step (b) of the method according to the first aspect. It is referred thereto as the same disclosure applies here.

In step (c), the separated hybrids are washed with the hybridization solution according to the third aspect. Details and preferred embodiments of the respective hybridization solution are described above and it is referred to the respective disclosure which also applies here. The components of the hybridization solution can be added a single solution for washing (what is preferred) or may be added separately in any order to the hybrids to generate the hybridization solution used for washing. The hybridization solution may also be diluted with an appropriate dilution solution such as e.g. water or other solvent what is preferred. The same ratios of hybridization solution: dilution solution can be used as are described above in the method according to the first aspect for the ratio hybridization solution: sample (or diluted sample). According to one embodiment, hybridization conditions are established during washing and hence are used in the washing composition which comprises the hybridization solution and the hybrids, which correspond to the hybridization conditions described above for the hybridization composition of the method according to the first aspect. It is referred to the respective disclosure which also applies here. Non limiting embodiments are desired in the following:
Subsequently, suitable and preferred embodiments of the washing composition and the hybridization solution suitable to establish these washing conditions are disclosed. The washing composition is established due to the addition of the hybridization solution and optionally a dilution solution.

When describing and defining the concentration of components "of the hybridization solution" in the washing composition, the volume contributed by the capture probe and the solid support is not considered in the determination of the concentration of the subsequently described components in the washing composition. According to one embodiment, the hybridization solution comprises its components in a concentrated form that allows it to be diluted with a dilution solution so as to achieve the proper final concentration in the washing composition that is used to wash the hybrids.

The hybridization solution used for washing and hence also the washing composition comprises a sodium salt. This also encompasses the use of a mixture of different sodium salts as "a" sodium salt. The sodium salt promotes binding of the poly(A) nucleic acids to the capture probe thereby reducing the risk that poly(A) nucleic acids are lost during washing. It may be an anorganic or organic sodium salt. According to one embodiment, the sodium salt is a sodium halide. According to one embodiment, the sodium salt is not a chaotropic salt. Preferably, the sodium halide is sodium chloride.

With respect to the desired reduction of non-poly(A) nucleic acid contaminations, it was found advantageous to reduce the concentration of the sodium salt in the washing composition. Thus, according to one embodiment, the washing composition comprises the sodium salt of the hybridization solution in a concentration ≤ 250 mM. The washing composition may comprise the sodium salt of the hybridization solution in a concentration selected from 25 mM to 250 mM, 35 mM to 200 mM, 40 mM to 175 mM, 50 mM to 150 mM, 55 mM to 125 mM, 60 mM to 115 mM and 60 mM to 100 mM.

According to a preferred embodiment, the washing composition comprises the sodium salt of the hybridization solution in a concentration that is ≤ 200 mM, ≤ 175 mM, ≤ 150 mM, ≤ 125 mM or ≤ 100 mM. Such lower concentrations of the sodium salt in the washing composition are preferred, as they provide stringent conditions for specific hybridizing the poly(A) nucleic acids to the capture probe, whereas unspecifically hybridized of non-poly(A) nucleic acids are washed away.

The hybridization solution and hence the washing composition comprises a quaternary ammonium salt. This also encompasses the use of a mixture of different quaternary ammonium salts as "a" quaternary ammonium salt. According to one embodiment, the quaternary ammonium salt is a tetraalkylammonium salt. The tetraalkylammonium salt may be a tetramethylammonium salt (TMA) or a tetraethylammonium salt (TEA). Suitable tetraalkylammonium salts include but are not limited to tetraethylammonium chloride (TEAC), tetramethylammonium chloride (TMAC), tetraethylammonium nitrate (TEAN), tetramethylammonium nitrate (TMAN), tetraethylammonium bromide (TEAB) and tetramethylammonium bromide (TMAB). According to one embodiment, the quaternary ammonium salt is not tetramethylammonium sulfate. Preferably, tetramethylammonium bromide is used as quaternary ammonium salt.

According to one embodiment, the washing composition comprises the quaternary ammonium salt of the hybridization solution in a concentration ≤ 3 M, ≤ 2.5 M, ≤ 2 M or ≤ 1.5 M. The washing composition may comprise the quaternary ammonium salt in a concentration ≥ 100 mM, ≥ 125 mM, ≥ 250 mM or ≥ 375mM. Preferably, the washing composition comprises the quaternary ammonium salt in a concentration selected from 0.1 M to 1.75M, 0.125 M to 1.5 M, 0.25 M to 1.25 M, 0.375 M to 1 M and 0.375 M to 0.75 M. As described above, the quaternary ammonium salt preferably is a tetraalkylammonium salt and suitable examples are described above.

According to one embodiment, the hybridization solution used for establishing the conditions in the washing composition comprises the quaternary ammonium salt in a concentration ≤ 6 M, ≤ 5 M, ≤ 4 M or ≤ 3 M. Said hybridization solution may comprise the quaternary ammonium salt in a concentration ≥ 200 mM, ≥ 250 mM, ≥ 500 mM or ≥ 750 mM. It may comprise the quaternary ammonium salt in a concentration selected from 0.2 M to 3.5 M, 0.25 M to 3M, 0.5 M to 2.5 M, 0.75 M to 2 M and 0.75 M to 1.5 M.

Suitable concentrations of the sodium salt and the quaternary ammonium salt can also be determined by the skilled person following the teachings provided herein.

Non-limiting preferred embodiments of the washing composition, in particular with respect to the contained sodium salt, which preferably is sodium chloride, and the quaternary ammonium salt, which preferably is a tetraalkylammonium salt, are described in the following. As discussed above, when describing the concentration of the components of the hybridization solution in the washing composition, the volume contributed by the capture probe and means used to assist the separation such as the solid support (if used for assisting the capturing and separation) is not considered in the determination of the concentration of the described components in the washing composition. Furthermore, as described, the components of the hybridization solution can be added as single solution to the sample or may be added separately in any order to the separated hybrids, e.g. using two or more solutions comprising at least one chemical of the hybridization solution to generate the hybridization solution that is used for washing.

According to one embodiment, the washing composition comprises the sodium salt of the hybridization solution in a concentration ≤ 250 mM and a tetraalkylammonium salt as quaternary ammonium salt. According to one embodiment, the washing composition comprises the sodium salt of the hybridization solution in a concentration selected from 25 mM to 175 mM, 50 mM to 150 mM, 55 mM to 125 mM. 60 mM to 115 mM and 60 mM to 100 mM and a tetraalkylammonium salt as quaternary ammonium salt in a concentration selected from 0.25M to 1.25M, 0.375 M to 1 M and 0.375 M to 0.75 M salt.

According to one embodiment, the washing composition comprises the sodium salt of the hybridization solution in a concentration selected from 37.5 mM to 125 mM, 50 mM to 100 mM and 55 mM to 87.5 mM and 60 mM to 100 mM and a tetraalkylammonium salt as quaternary ammonium salt in a concentration selected from 0.25M to 1.25M, 0.375 M to 1 M and 0.375 M to 0.75 M, wherein the sodium salt is sodium chloride.

As described, the hybridization conditions according to the invention that can also be used to establish stringent washing conditions are based on a balanced combination of a sodium salt and a quaternary ammonium salt which result in an efficient isolation of poly(A) nucleic acids while preventing carry-over on non-poly(A) nucleic acids. Therefore, the hybridization solution and respectively the washing composition does not contain other hybridization promoting salts besides the sodium salt and the quaternary ammonium salt in a concentration that would counteract these advantageous effects. Therefore, according to embodiments, the hybridization solution and/or washing composition does not contain hybridization promoting salts such as lithium or potassium chloride or other non-sodium halides, MgCl₂ and/or chaotropic salts in a concentration that would counteract the advantageous effects achieved by the combination of the sodium salt and the quaternary ammonium salt. In embodiments, the concentration of such salts if at all present, is 100mM or less, 75mM or less, 50mM or less or 25mM or less. Preferably, the hybridization solution does not contain any hybridization promoting salts besides the sodium salt and the quaternary ammonium salt.

In step (d), poly(A) nucleic acids are released from the washed hybrids. Details with respect to release step (d) have also been described above in conjunction with the method according to the first aspect. It is referred to the above disclosure, which also applies here.

Using the washing conditions of the present disclosure at least in one washing step of a poly(A) nucleic acid isolation procedure has the advantage that non- poly(A) nucleic acids that may have bound in the hybridization step (a) (e.g. if the hybridization conditions of the present invention have not been used) can be subsequently washed away. One or more further washing steps can be performed in addition. This is also preferred in order to improve the purity of the poly(A) nucleic acids and to remove components of the hybridization solution that may potentially interfere with downstream applications.

As described above, the poly(A) nucleic acid preferably is poly(A) RNA. It is referred to the above disclosure made in conjunction with the method according to the first aspect which also applies here.

This invention is not limited by the exemplary methods and materials disclosed herein, and any methods and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

As used in the subject specification and claims, the singular forms "a", "an" and "the" include plural aspects unless the context clearly dictates otherwise. Thus, for example, reference to "a sodium salt" includes a single type of sodium salt, as well as two or more sodium salts. Likewise, reference to a "quaternary ammonium salt", a "capture probe", "detergent", a "buffering agent" and the like includes single entities and combinations of two or more of such entities. Reference to "the disclosure" and "the invention" and the like includes single or multiple aspects taught herein; and so forth. Aspects taught herein are encompassed by the term "invention".

The term "solution" as used herein in particular refers to a liquid composition, preferably an aqueous composition. It may be a homogenous mixture of only one phase but it is also within the scope of the present invention that a solution comprises solid constituents such as e.g. precipitates.

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### EXAMPLES

The examples are for illustrative purpose only and are not to be construed as limiting this invention in any manner.

### Materials and methods

### Isolation of poly(A) nucleic acids

For the experiments, total RNA isolated from Jurkat cells was used as nucleic acid containing sample from which poly(A) RNA was isolated. As capture probe, dC₁₀T₃₀ capture oligonucleotides covalently linked to the surface of (non-magnetic) polystyrene-latex particles (Oligotex suspension; QIAGEN) or dT₁₄ capture oligonucleotides covalently linked to the surface of magnetic particles (Seradyn Magnetic Beads) were used.

### Basic Protocol

The performed poly(A) nucleic acid isolation protocol was based on the Oligotex® handbook protocol (Qiagen). The main steps of the Basic Protocol are summarized in the following:
- Addition of the nucleic acid containing sample (total RNA) into an RNase-free reaction tube and adjusting the volume to 250µl with RNase free water.
- Addition of 250µl hybridization solution.
- Addition of the capture oligonucleotides immobilized to a solid support (15µl Oligotex suspension (Qiagen) or 25µl functionalized Seradyn Magnetic Beads if not indicated otherwise).
- The contents are mixed and incubated for 3 min at 70°C to denature the RNA.
- Incubation for 10 min at 20°C - 30°C to allow hybridization of the poly(A) RNA to the capture oligonucleotides.
- Separation of the formed hybrids from the remaining sample. The separation process depends on the type of solid support used. In case of Oligotex particles, the sample was centrifuged to pellet the Oligotex/poly(A) RNA complexes and the supernatant (remaining sample) was discarded. In case of magnetic particles, the magnetic particles/poly(A) RNA complexes were pelleted with the aid of a magnetic field and the supernatant (remaining sample) was discarded. Alternatively, separation may also occur using spin columns.
- Resuspension of the pelleted complexes in 400µl wash buffer (10 mM Tris-Cl, pH 7.5; 150 mM NaCl; 1 mM EDTA) by vortexing. In case of Oligotex particles, the resuspended sample was added to a spin column and centrifuged. In case of magnetic particles, the magnetic particles were pelleted with the aid of a magnetic field and the supernatant was discarded. Alternatively, separation may also occur using spin columns.
- Addition of 400µl wash buffer (see above) to the spin column and centrifugation (Oligotex particles) or addition of 400µl wash buffer (see above) to the pelleted complexes and resuspension by vortexing followed by magnetic separation (magnetic particles) (alternatively, separation may also occur using spin columns).
- Release of the poly(A) RNA from the washed hybrids by addition of 50µl elution buffer (5 mM Tris-Cl, pH 7.5) (70°C) and resuspension by pipetting up and down 3-4 times.
- Separation of the poly(A) containing eluate from the solid support by centrifugation (Oligotex particles) or magnetic separation (magnetic particles) and recovery of the eluate (flow-through in case of Oligotex particles; supernatant in case of magnetic particles; however, also with magnetic particles spin columns may be used).

### Magnetic Protocol

Alternatively, a further magnetic particle poly(A) nucleic acid isolation protocol (Magnetic Protocol) was followed. In brief, total RNA (no dilution) was contacted with 370µl hybridization solution and 50µl magnetic particles (see above). Hybridization occurred at room temperature for 5min. After magnetic separation of the magnetic particles with the bound poly(A) nucleic acids, the particles were washed several times and the poly(A) nucleic acids were eluted with a low salt buffer.

### Real time RT-PCR analysis

RNA detection was performed using SYBR Green reporter dye based quantitative real time RT-PCR assays. The following target RNAs were detected: 18S rRNA (non-poly(A) contamination), 28S rRNA (non-poly(A) contamination) and also other rRNAs (non-poly(A) contamination) and RPL (60S ribosomal protein L12 gene) mRNA, PPIA (peptidylprolyl isomerase A gene) mRNA, CDH2 (cadherin-2 gene) mRNA and/or GAPDH (glyceraldehyde 3-phosphate dehydrogenase gene) mRNA.

### Tested hybridization conditions

### 1. Comparative example 1

Example 1 demonstrates that the nature of the salt used for hybridization is important. The performed hybridization studies with LiCl, MgCl₂ and KCI containing hybridization solutions showed only a minor or no influence on non-poly(A) recovery (as determined by analysis of rRNA content in the eluates) if these salts were used in different concentrations. Different settings were tested:

### a) Influence of reducing the LiCl concentration in the hybridization solution

The effect of decreasing concentrations of LiCl (700 mM, 500 mM, 300 mM) in the hybridization solution on rRNA depletion and poly(A) mRNA enrichment was tested by analyzing 18S rRNA and RPL transcript levels in the eluates obtained after poly(A) enrichment. The 18S rRNA and RPL transcript levels of the initial total RNA sample were analyzed in parallel as reference control.

5µg total RNA from Jurkat cells served as starting material for the poly(A) RNA isolation procedure which was performed according to the Magnetic Protocol. The tested hybridization solutions contained a Tris-buffer (pH 7.5), an anionic detergent (LiDs) and LiCl in different concentrations (300 mM, 500 mM or 700 mM).

Two washing buffers, namely washing buffer 1 (150mM LiCl, LiDs) and 2 (150mM LiCl) were used.

In brief, poly(A) RNA isolation was performed manually as follows:
- Pipetting 7.4µl total RNA (680ng/µl) into an RNase-free tube. Addition of 370µl hybridization solution.
- Addition of 50µl magnetic beads with immobilized oligo d(T) capture oligonucleotide and mixing the contents by flicking the tube.
- Incubation for 5min at room temperature to allow hybridization of the poly(A) RNA to the capture oligonucleotide.
- Applying a magnet field for 1 min to separate the magnetic bead:poly(A) RNA complexes from the remaining sample.
- 2 x washing: addition of 300µl wash buffer 1 for each washing step to the magnetic beads:poly(A) RNA pellet and resuspension by vortexing; applying a magnet field for 1 min; discard supernatant.
- 3 x washing: addition of 300µl wash buffer 2 for each washing step to the magnetic beads:mRNA pellet and resuspension by vortexing; applying a magnet field and incubation for 1 min; discard supernatant.
- Addition of 50µl elution solution (low salt solution, neutral pH); incubation for 2 min at 65°C; applying a magnet field and incubation for 1 min.
- Recovery of the supernatant containing the eluted poly(A) RNA and transfer to another RNase-free tube.

For the real time RT-PCR assays for detecting the 18S rRNA and RPL mRNA targets, the obtained eluates were diluted 1:280 (2µl eluate + 558µl H2O). Initial total RNA (680ng/µl) was diluted 1:1838,27 to receive a concentration of 0.37 ng/µl. 5µl of diluted pol(A) RNA eluate or diluted total RNA was used in the real time RT-PCR analysis. All approaches were analyzed in duplicates per condition.

### Results

The mean Ct value was calculated from two parallel repetitions per approach. The Ct value, abbreviated for cycle threshold, corresponds to that PCR cycle number at which the fluorescence of the SYBR Green reporter dye first rises exponentially above the background value (threshold). The lower the Ct value, the more cDNA template and thus the more initial transcript was present in the eluate. An increase in the Ct value of a transcript indicates that some transcript was lost during the poly(A) RNA enrichment. Table 1 shows the results.

**Table 1: The mean Ct values of the 18S rRNA and the RPL mRNA as determined by real time RT-PCR analyses are shown as obtained using decreasing concentrations of LiCl in the hybridization solution.**

| | **18S rRNA** | | **RPL mRNA** | |
|---|---|---|---|---|
| **Hybridization Solution** | **Mean Ct value** | **Std. Deviation** | **Mean Ct value** | **Std. Deviation** |
| **total RNA** | 7.97 | 0.85 | 20.99 | 0.00 |
| **700mM LiCl** | 13.09 | 0.05 | 21.71 | 0.04 |
| **500mM LiCl** | 13.10 | 0.02 | 21.71 | 0.03 |
| **300mM LiCl** | 13.27 | 0.10 | 21.73 | 0.09 |

All tested hybridization solutions depleted 18S rRNA transcript levels, as can be seen from the significantly increased mean Ct value compared to the Ct value obtained with the initial total RNA sample. The 18S rRNA and RPL mRNA levels in the eluate are comparable among the different hybridization solutions tested. Thus, increasing the hybridization stringency by reducing the LiCl salt concentration in the hybridization solution and hence in the hybridization composition had no influence on rRNA depletion or mRNA enrichment.

### b) Influence of reducing the KCl or MgCl₂ concentration in the hybridization solution

Furthermore, the effects on rRNA depletion (based on 18S rRNA) and poly(A) mRNA enrichment (based on PPIA and CDH2 transcript levels) was tested when decreasing the concentration of KCI or MgCl₂ in the hybridization solution. The hybridization solution comprised besides water and KCI or MgCl₂ in different concentrations (for each salt: 1 M, 500 mM or 100 mM), 20mM Tris, 2% SDS and 2.5mM EDTA; the pH value was adjusted to 7.5 with HCl or NaOH. A corresponding hybridization solution comprising 1 M NaCl instead of KCI or MgCl₂ was tested as reference standard.

5µg total RNA purified from Jurkat cells served as starting material. For poly(A) RNA isolation, the Basic Protocol as described above under Materials and Methods was followed using as capture oligonucleotide functionalized solid support an Oligotex suspension and spin columns to assist separation.

For real time RT-PCR analysis, the obtained eluates were diluted 1:200. Initial total RNA (1351ng/µl) was diluted 3.7µg/46,3µl RNase free water; from this initial dilution a 1:200 dilution was obtained. 5µl of diluted poly(A) RNA eluate or diluted total RNA was used in the real time RT- PCR analysis.

### Results

The mean Ct value was determined and the results are shown in Table 2.

**Table 2: The mean Ct values of the 18S rRNA and of the PPIA and CDH2 mRNA as determined by real time RT-PCR analyses is shown as obtained using decreasing concentrations of KCI or MgCl₂ or 1 M NaCl in the hybridization solution.**

| | **18s rRNA** | **PPIA mRNA** | **CDH2 mRNA** |
|---|---|---|---|
| **Hybridization Solution** | **Mean Ct value** | **Mean Ct value** | **Mean Ct value** |
| **total RNA** | 7.01 | 18.78 | 25.51 |
| **1 M NaCl** | 14.91 | 21.53 | 27.81 |
| **1 M KCl** | 15.91 | 22.62 | 28.85 |
| **500 mM KCl** | 15.48 | 22.45 | 28.95 |
| **100 mM KCl** | 16.81 | 23.84 | 30.45 |
| **1 M MgCl₂** | 17.55 | 28.25 | 34.57 |
| **500 mM MgCl₂** | 15.66 | 26.17 | 33.70 |
| **100 mM MgCl₂** | 14.49 | 21.61 | 28.08 |

All tested hybridization solutions depleted 18S rRNA transcript levels, as can be seen from the increased mean Ct value compared to the Ct value obtained with the initial total RNA sample. Decreasing the KCI salt concentration in the hybridization solution leads to no clear results regarding a potential improvement on rRNA depletion. A reduction of the KCI salt from 1 M to 500 mM in the hybridization solution resulted in a lower rRNA depletion efficiency compared to the 1 M KCI approach while the hybridization solution comprising 100 mM KCI depleted more rRNA than the 1 M KCL containing hybridization buffer. A reduction of the MgCl₂ concentration in the hybridization solution is negatively correlated with 18S rRNA depletion. 1 M MgCl₂ in the hybridization solution achieved the best rRNA depletion but was associated with significant losses in the target mRNA. The lowest tested MgCl₂ concentration (100 mM) shows only similar good rRNA depletion and mRNA enrichment efficiency as the hybridization solution comprising 1 M NaCl.

### 2. Comparative example 2

The effects on rRNA depletion (based on 18S and 28S rRNA) and poly(A) mRNA enrichment (based on GPDH and PPIA mRNA) was analysed when decreasing the concentration of NaCl in the hybridization solution. The hybridization solution comprised besides water and NaCl in different concentrations (1 M, 400 mM, 300 mM, 200 mM, 100 mM or 50mM), 20mM Tris (pH 7.5), 2% SDS and 2.5mM EDTA (pH 8).

For poly(A) RNA isolation, the Basic Protocol as described above under Materials and Methods was followed using as capture oligonucleotide functionalized solid support an Oligotex suspension and spin columns to assist separation. 5µg total RNA purified from Jurkat cells served as starting material.

For real time PCR assays against the rRNA and mRNA targets, the obtained eluates were diluted 1:1000. Initial total RNA (1.27µg/µl) was diluted by adding 3.94µl RNA to 46.06µl RNase free water. 5µl of diluted poly(A) RNA eluate or diluted total RNA was used in the real time RT- PCR analysis.

### Results

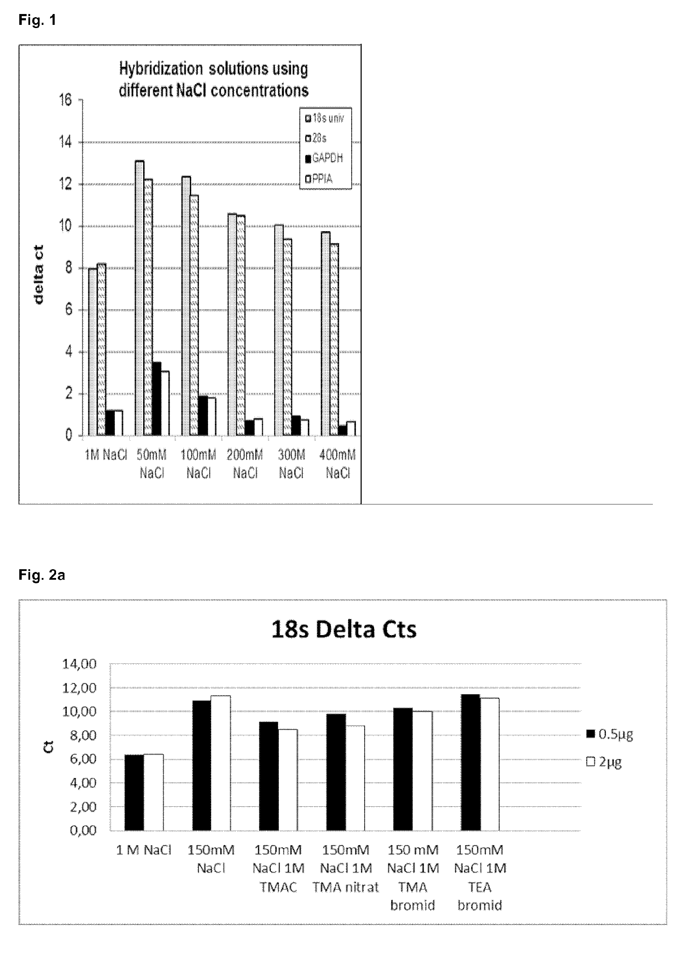

The mean Ct value was determined and the results are shown in Table 3. Figure 1 shows the delta Ct values of the tested conditions (delta Ct value calculation: mean Ct value determined after poly(A) RNA enrichment minus the mean CT value determined for the initial total RNA sample). All tested hybridization solutions depleted 18S rRNA during poly(A) enrichment, as can be seen from the significantly increased mean Ct value compared to the mean Ct value obtained with the initial total RNA sample.

**Table 3: The mean Ct values of the 18S rRNA, 28S rRNA and of the GAPDH and PPIA mRNA as determined by real time RT-PCR analyses is shown as obtained using decreasing concentrations of NaCl in the hybridization solution.**

| | **18S rRNA** | **28S rRNA** | **GAPDH mRNA** | **PPIA mRNA** |
|---|---|---|---|---|
| **Hybridization Solution** | **Mean Ct value** | **Mean Ct value** | **Mean Ct value** | **Mean Ct value** |
| **total RNA** | 5.66 | 7.40 | 16.63 | 18.34 |
| **1M NaCl** | 13.64 | 15.58 | 17.85 | 19.56 |
| **400mM NaCl** | 15.35 | 16.55 | 17.09 | 19.01 |
| **300mM NaCl** | 15.71 | 16.75 | 17.58 | 19.11 |
| **200mM NaCl** | 16.24 | 17.88 | 17.37 | 19.16 |
| **100mM NaCl** | 18.02 | 18.83 | 18.52 | 20.14 |
| **50mM NaCl** | 18.76 | 19.62 | 20.14 | 21.39 |

All tested hybridization solutions depleted 18S rRNA transcript levels, as can be seen from the significantly increased mean Ct value compared to the Ct value obtained with the initial total RNA sample. An enormous depletion of rRNA levels was observed in a concentration dependent manner. The lower the NaCl concentration in the hybridization solution, the lower the amount of rRNA contamination in the eluates and accordingly, the better the rRNA depletion efficiency. The highest rRNA depletion was achieved with NaCl concentrations below 200mM in the hybridization solution as can be derived from the significantly increased Ct values. The reduction of NaCl concentration in the hybridization solution had, however, a less severe impact on mRNA recovery. Thus, in relative terms, significantly more rRNA was depleted due to the more stringent hybridization conditions than was target mRNA lost. Thus, reducing the NaCl concentration in the hybridization solution showed a dramatic effect on 18S and 28S rRNA recovery and only some effect on mRNA recovery. This trend is also evident from the results shown in Fig. 1. However, even though the non-poly(A) RNA recovery was more severely affected, decreasing the concentration of NaCl below 200 mM still resulted in that mRNA was lost during these hybridization conditions as can be seen from the increased Ct values of the mRNA targets.

Furthermore, in additional experiments, the amount of SDS was varied (0%, 0.2% and 2%). This variation had no effect on the hybridization, i.e. rRNA depletion and mRNA recovery remained approximately the same (data not shown).

### 3. Example 3

In example 3, the effect on rRNA depletion and poly(A) mRNA enrichment utilizing a low ionic strength NaCl salt concentration (150 mM) in combination with different quaternary ammonium salts in the hybridization solution was analyzed. The tested hybridization solutions had the following composition: 20mM Tris, 150mM NaCl, 0.2% SDS, EDTA, water and 1M of the tested quaternary ammonium salt. As quaternary ammonium salt, the tetraalkylammonium salts tetramethylammonium chloride (TMAC), tetramethylammonium nitrate (TMA nitrate), tetramethylammonium bromide (TMA bromide) or tetraethylammonium bromide (TEA bromide) were tested. The poly(A) RNA isolation procedure was performed according to the Basic Protocol described above under Materials and Methods using as capture oligonucleotide functionalized solid support Seradyn Magnetic Beads.

Total RNA (0.5µg and 2µg) from Jurkart cells served as starting material. The rRNA depletion efficiency was tested based on 18S rRNA and 28S rRNA levels and poly(A) RNA enrichment based on PPIA and GAPDH transcripts.

For real time RT-PCR assays against the rRNA and mRNA targets, the obtained eluates were diluted 1:200. Initial total RNA (785.5ng/µl) was diluted by adding 6.37µl to 43.63µl RNase free water. From this initial dilution a 1:200 dilution was obtained. 5µl of diluted poly(A) RNA eluate or diluted total RNA was used in the real time RT- PCR analysis. The mean Ct value for the 18S rRNA and 28S rRNA and GAPDH mRNA and PPIA mRNA per condition using 0.5 µg or 2 µg total RNA as starting material was calculated from two parallel experiments per condition.

### Results

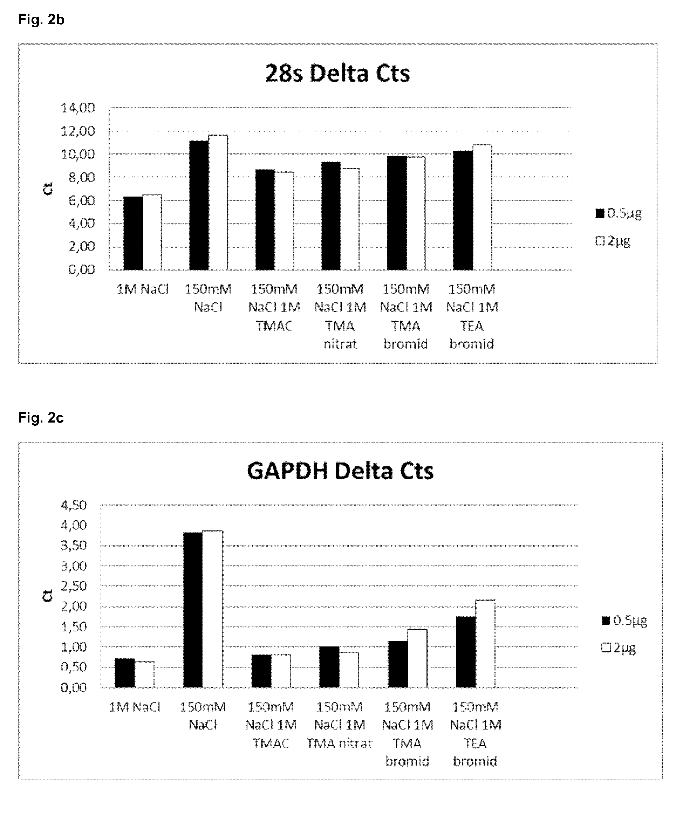

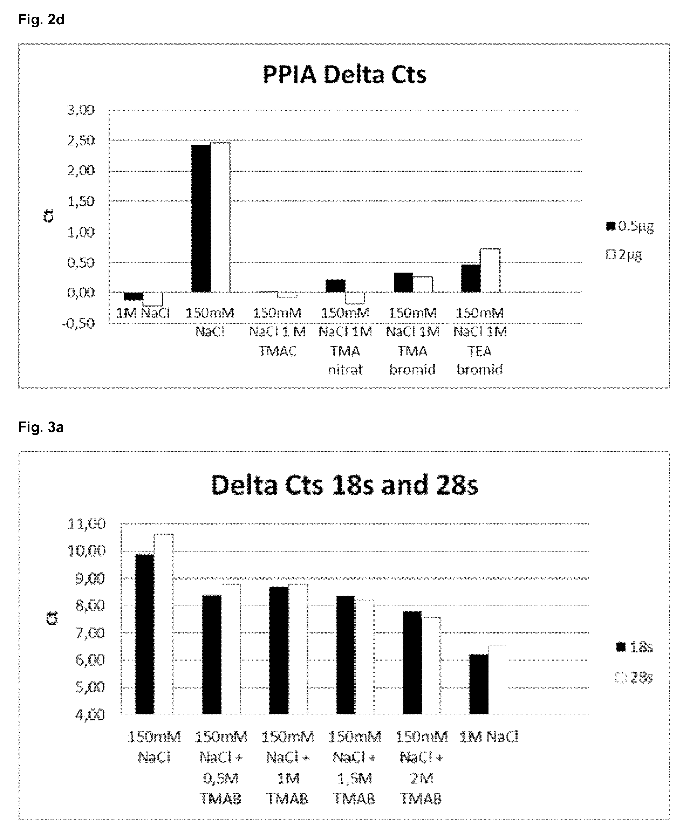

Figure 2 a) to d) shows the delta Ct values (calculated as explained above) obtained with the tested conditions. As can be seen, the effect on rRNA depletion and mRNA recovery is quite comparable when using 0.5 µg or 2.0 µg total RNA as starting material for poly(A) RNA isolation. All tested hybridization solutions comprising 150 mM NaCl in combination with a quaternary ammonium salt achieved significantly higher 18S rRNA (Figure 2 a) and 28S rRNA (Figure 2 b) depletion rates compared to the 1 M NaCl reference hybridization solution. Furthermore, as can be seen from Figure 2 c) and d), the hybridization solutions wherein 150mM NaCl was used in combination with a tetraalkylammonium salt showed significantly less unwanted losses in the poly(A) RNA compared to the 150 mM NaCl containing hybridization solution as determined based on the target mRNAs PPIA and GAPDH. Therefore, mRNA losses could be significantly reduced compared to the hybridization solution not containing a tetraalkylammonium salt.

Thus, combining the reduction of the NaCl concentration in the hybridization solution (and hence the hybridization composition) with the addition of a quaternary ammonium salt results in advantageous hybridization conditions, wherein unwanted rRNA was efficiently depleted, while the wanted poly(A) RNA was efficiently hybridized to the capture oligonucleotides and thus enriched. Therefore, the hybridization conditions taught by the present invention wherein a lower concentration of a sodium salt such as sodium chloride is used in combination with a quaternary ammonium salt provide an advantageous balance between non-poly(A) depletion and poly(A) RNA recovery.

### 4. Example 4

Here, different concentrations of TMA bromide in the hybridization solution were used and the effects on rRNA depletion and poly(A) mRNA enrichment was analyzed. The tested hybridization solutions had the following composition: 20mM Tris, 150mM NaCl, 0.2% SDS and EDTA, water and 0.5 M, 1 M, 1.5 M or 2 M TMAB. As reference, corresponding hybridization solutions comprising 1 M or 150 mM NaCl but no quaternary ammonium salt were tested in parallel.

The poly(A) RNA isolation procedure was performed according to the Basic Protocol described above under Materials and Methods using as capture oligonucleotide functionalized solid support Seradyn Magnetic Beads. 2µg total RNA isolated from Jurkart cells served as starting material. The rRNA depletion efficiency was tested based on 18S rRNA and 28S rRNA levels and poly(A) RNA enrichment based on PPIA and GAPDH mRNA.

For real time RT-PCR assays against the rRNA and mRNA targets, the obtained eluates were diluted 1:200. Initial total RNA (0.97µg/µl) was diluted by adding 2.06µl to 47.95µl RNase free water. From this initial dilution a 1:200 dilution was obtained. 5µl of diluted poly(A) RNA eluate or diluted total RNA was used in the real time RT- PCR analysis.

### Results

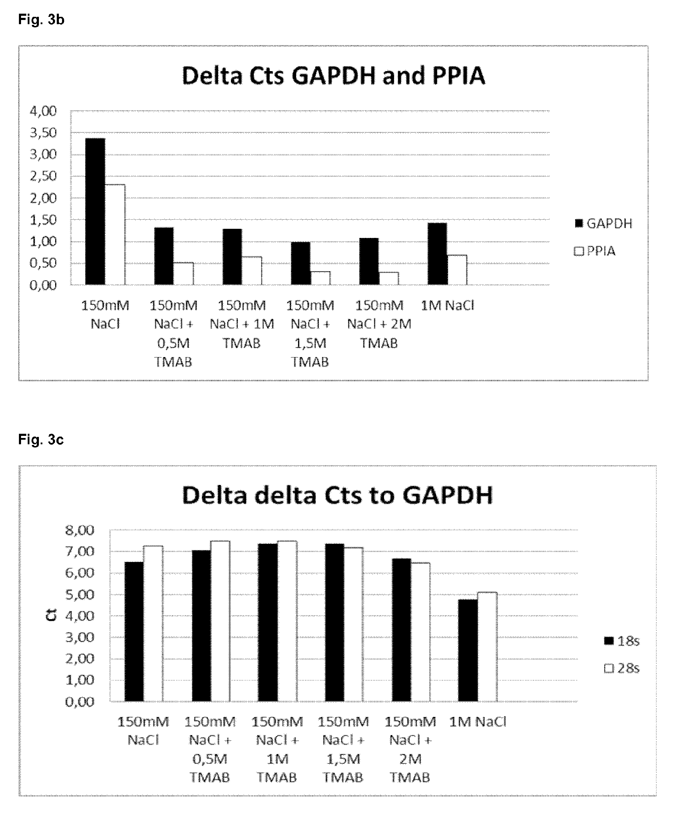

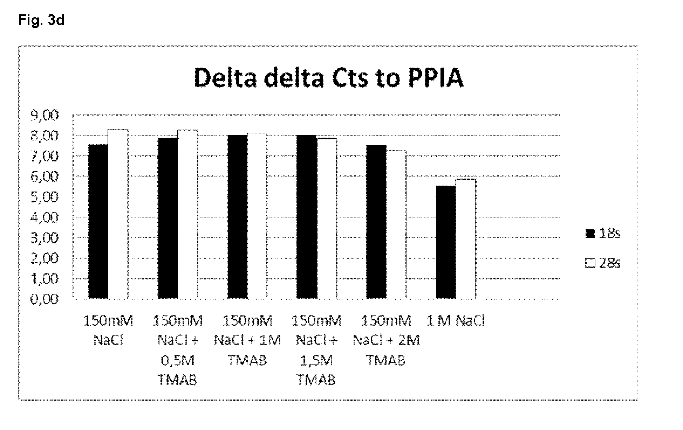

Figure 3 a) to 3 d) show the results:
Figure 3a) shows the delta Ct values obtained for 18S and 28S rRNA. As can be seen, the rRNA depletion results were improved compared to the 1 M NaCl reference hybridization solution. Figure 3b) shows the delta Ct values obtained for the GAPDH and PPIA target mRNAs. As can be seen, the results obtained with the hybridization solutions according to the invention were improved compared to the 150mM and 1 M NaCl reference hybridization solutions under all tested conditions.
Figure 3 c) shows the delta delta Ct values to GAPDH and Figure 3d) the delta delta Ct values to PPIA. Delta delta Ct values were calculated from delta Ct values (mean CT values of conditions normalized against the corresponding initial total RNA control) according to the following formula: delta Ct value of 18S rRNA or 28S rRNA at a respective condition minus the delta Ct value of GAPDH mRNA levels or PPIA mRNA levels at a respective condition. A higher delta delta Ct value is advantageous as it demonstrates high rRNA depletion rates. As can be seen, the results were improved compared to the hybridization solution containing 1 M NaCl. Furthermore, similar or better delta delta Ct values were obtained with the hybridization solutions according to the invention compared to the hybridization solution containing 150mM NaCl. Therefore, compared to the hybridization solution containing 150 mM salt, the delta delta Ct results were maintained or even improved and, importantly, the mRNA recovery was significantly improved with the hybridization solutions of the invention as is demonstrated by Figs. 3 a) and b).

Example 4 thereby clearly demonstrates the benefits of the hybridization solution and hybridization composition according to the invention and shows that the tetraalkylammonium salt can be used in different concentrations. These results were also confirmed in other experiments wherein corresponding hybridization solutions comprising 0.2 M TMAB were tested. Thus, the quaternary ammonium salt works in high and low concentrations.

### 5. Example 5

The poly(A) RNA isolation procedure was performed according to the Basic Protocol described above under Materials and Methods using as capture oligonucleotide functionalized solid support Seradyn Magnetic Beads. The hybridization solution comprised 20mM Tris, 150mM NaCl, 0.2% SDS water and 1 M TMAB. 5µg total RNA isolated from Jurkart cells served as starting material. The residual amount of different rRNAs (5S rRNA, 5.8S rRNA, 12s rRNA, 16S rRNA, 18S rRNA and 28S rRNA) was determined in the isolated poly(A) RNA eluate based on real time RT-PCR analysis. 7 samples were evaluated. The obtained eluates were diluted 1:1000. Initial total RNA was diluted to 5.1µg/50µl. From this initial dilution a 1:1000 dilution was obtained. Further 1:10 dilutions were then prepared. 5µl of diluted poly(A) RNA eluate or diluted total RNA was used in the real time RT- PCR analysis.

### Results

**Table 4: residual rRNA in the eluates after poly(A) RNA isolation**

| **5S rRNA** | **Residual rRNA** |
|---|---|
| Poly(A) RNA eluate | 0.00% |
| Control | 100% |

| **5,8S rRNA** | **Residual rRNA** |
|---|---|
| Poly(A) RNA eluate | 0.00% |
| Control | 100% |

| **12S rRNA** | **Residual rRNA** |
|---|---|
| Poly(A) RNA eluate | 0.22% |
| Control | 100% |

| **16S rRNA** | **Residual rRNA** |
|---|---|
| Poly(A) RNA eluate | 0.91% |
| Poly(A) RNA eluate | 1.27% |
| Control | 100% |

| **18S rRNA** | **Residual rRNA** |
|---|---|
| Poly(A) RNA eluate | 0.00% |
| Control | 100% |

| **28s rRNA** | **Residual rRNA** |
|---|---|
| Poly(A) RNA eluate | 0.00% |
| Control | 100% |

As can be seen, different types of rRNA were efficiently depleted usually ≥ 99% and even to 100%. This demonstrates the extraordinary depletion efficiency that is achieved using the hybridization conditions of the invention.

### 6. Comparative example 6

In example 6, the effect of different substances (DMSO, formamide, TMAC, betaine) added to a hybridization solution containing 500mM LiCl was tested. 5 µg total RNA isolated from Jurkart cells served as starting material for poly(A) nucleic acid enrichment using the Magnetic Protocol described above. The hybridization composition was prepared by mixing 4.20µl total RNA, 370µl hybridization solution and 50µl magnetic particles functionalized with the capture oligonucleotide.

Reference LiCl buffer: (Tris pH: 7.5, 500mM LiCl, 1% anionic detergent)
LiCI - DMSO: composition as reference LiCl buffer + DMSO (final: 5%)
LiCI - Formamide: composition as reference LiCl buffer + formamide (final: 2%)
LiCI - TMAC: composition as reference LiCl buffer + TMAC (final: 100 nM)
LiCI - Betaine: composition as reference LiCl buffer + betaine (final: 1 M)

### Results

Table 5 shows the result of the quantitative real time RT-PCR analysis. The delta CT values were determined for 18S and 28S rRNA to analyse non-poly(A) depletion and GAPDH and CDH2 to analyse mRNA enrichment.

**Table 5 Delta Ct values of the 18S rRNA and 28S rRNA and of the GAPDH and CDH2 mRNA are shown.**

| | **18S rRNA** | **28S rRNA** | **GAPDH mRNA** | **CDH2 mRNA** |
|---|---|---|---|---|
| **Hybridization solution** | **delta Ct** | **delta Ct** | **delta Ct** | **delta Ct** |
| LiCl | 5.22 | 5.72 | 0.83 | 0.44 |
| LiCl-DMSO | 4.98 | 6.00 | 0.83 | 0.30 |
| LiCl-Formamide | 5.24 | 5.72 | 0.94 | 0.32 |
| LiCl-TMAC | 5.07 | 5.66 | 0.83 | 0.53 |
| LiCl-Betaine | 5.25 | 5.86 | 0.90 | 0.48 |

Furthermore, the reference hybridization buffer (see above) was supplemented with different concentrations of TMAC (see table 6 below) to analyse whether higher TMAC concentrations could improve the results. However, in combination with LiCl, substantially no effect on mRNA recovery or rRNA depletion was seen. The example was repeated using different hybridization temperatures (40°C, 50°C and 60°C), which had no effect.

**Table 6: Delta Ct values of the 18S rRNA and 28S rRNA and of the GAPDH and CDH2 mRNA are shown.**

| **Hybridization solution** | **18S** | **28S** | **GAPDH** | **CDH2** |
|---|---|---|---|---|
| 1M TMAC | 12.49 | 12.90 | 17.59 | 23.39 |
| 100mM TMAC | 12.51 | 13.09 | 17.58 | 23.40 |
| 10mM TMAC | 12.97 | 13.20 | 18.19 | 23.62 |
| Reference (No TMAC) | 12.63 | 13.19 | 17.56 | 23.40 |

Taking the experiments described before into account this result validates that only the combination of the NaCl concentration in combination with a quaternary ammonium salt in the hybridization solution achieves a significant reduction of non-poly(A) RNA obtained after poly(A) RNA enrichment while keeping the mRNA bound on the oligonucleotide capture probe thereby ensuring effective and selective poly(A) RNA recovery.

## Claims

1. A method for isolating poly(A) nucleic acids having a single stranded poly(A) stretch from a nucleic acid containing sample comprising:
(a) providing a hybridization composition comprising:
i) a nucleic acid containing sample;
ii) a hybridization solution comprising:
aa. a sodium salt;
bb. a quaternary ammonium salt;
wherein the components of the hybridization solution can be added as single solution to the sample or may be added separately in any order to the sample;
iii) a capture probe capable of hybridizing to the poly(A) stretch of the poly(A) nucleic acids;
and incubating said hybridization composition under conditions to form nucleic acid-hybrids between the poly(A) nucleic acids and the capture probe;
(b) separating the formed hybrids from the remaining sample.

2. The method according to claim 1, wherein
a) the hybridization composition comprises the sodium salt of the hybridization solution in a concentration ≤ 250 mM, preferably in a concentration selected from 25 mM to 250 mM, 35 mM to 200 mM, 40 mM to 175 mM, 50 mM to 150 mM, 55 mM to 125 mM, 60 mM to 115 mM and 60 mM to 100 mM; and/or
b) the hybridization solution comprises the sodium salt in a concentration ≤ 500 mM, preferably in a concentration selected from 50 mM to 500 mM, 75 mM to 400 mM, 85 mM to 350 mM, 100 mM to 300 mM, 115 mM to 250 mM, 120 mM to 225 mM and 125 mM to 200 mM.

3. The method according to claim 1 or 2, wherein the quaternary ammonium salt is a tetraalkylammonium salt, preferably selected from tetramethylammonium salts (TMA) and tetraethylammonium salts (TEA).

4. The method according to claim 3, having one or more of the following characteristics:
a) the hybridization composition comprises the tetraalkylammonium salt of the hybridization solution in a concentration ≤ 1.5 M, preferably in a concentration selected from 0.1 M to 1.75 M, 0.125 M to 1.5 M, 0.25 M to 1.25 M, 0.375 M to 1 M and 0.375 M to 0.75 M; and/or
b) the hybridization solution comprises the tetraalkylammonium salt in a concentration ≤ 3 M, preferably in a concentration selected from 0.2 M to 2.5 M, 0.5 M to 2 M and 0.75 M to 1.5 M; and/or
c) the tetraalkylammonium salt is selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB; and/or
d) wherein the tetraalkylammonium salt preferably is tetramethylammonium bromide.

5. The method according to one or more of claims 1 to 4, comprising:
(a) providing a hybridization composition comprising:
i) a nucleic acid containing sample;
ii) a hybridization solution comprising:
aa. a sodium salt in a concentration ≤ 500 mM;
bb. a tetraalkylammonium salt as quaternary ammonium salt;
wherein the components of the hybridization solution can be added as single solution to the sample or may be added separately in any order to the sample;
iii) a capture probe capable of hybridizing to the poly(A) stretch of the poly(A) nucleic acids;
wherein the hybridization composition comprises the sodium salt of the hybridization solution in a concentration ≤ 250 mM,
and incubating said hybridization composition under conditions to form nucleic acid-hybrids between the poly(A) nucleic acids and the capture probe;
(b) separating the formed hybrids from the remaining sample.

6. The according to one or more of claims 1 to 5 comprising:
(a) providing a hybridization composition comprising:
i) a nucleic acid containing sample;
ii) a hybridization solution comprising:
aa. a sodium salt in a concentration selected from 50 mM to 350 mM, 75 mM to 300 mM, 100 mM to 250 mM and 125 mM to 200 mM;
bb. a tetraalkylammonium salt as quaternary ammonium salt in a concentration selected from from 0.2 M to 2.5 M, 0.55 M to 2 M and 0.75 M to 1.5 M;
wherein the components of the hybridization solution can be added as single solution to the sample or may be added separately in any order to the sample;
iii) a capture probe capable of hybridizing to the poly(A) stretch of the poly(A) nucleic acids;
wherein the hybridization composition comprises the sodium salt of the hybridization solution in a concentration selected from 25 mM to 175 mM, 37.5 mM to 150 mM, 50 mM to 125 mM and 62.5 mM to 100 mM and the tetraalkylammonium salt in a concentration selected from 0.1 M to 1.25 M, 0.25 M to 1 M and 0.375 M to 0.75 M,
and incubating said hybridization composition under conditions to form nucleic acid-hybrids between the poly(A) nucleic acids and the capture probe;
(b) separating the formed hybrids from the remaining sample.

7. The method according to one or more of claims 1 to 6, in particular claim 5 or 6, wherein the poly(A) nucleic acid is poly(A) RNA and wherein preferably, the nucleic acid containing sample is total RNA.

8. The method according to one or more of claims 1 to 7, in particular claims 5 to 7, wherein the sodium salt is sodium chloride.

9. The method according to claim 8, for isolating poly(A) RNA from a total RNA sample, comprising:
(a) providing a hybridization composition comprising:
i) a nucleic acid containing sample;
ii) a hybridization solution comprising:
aa. a sodium salt which is sodium chloride in a concentration selected from 75 mM to 250 mM, 100 mM to 200 mM and 125mM to 175mM;
bb. a tetraalkylammonium salt selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB) as quaternary ammonium salt in a concentration selected from from 0.2 M to 2.5 M, 0.5 M to 2 M and 0.75 M to 1.5 M;
wherein the components of the hybridization solution can be added as single solution to the sample or may be added separately in any order to the sample;
iii) a capture probe capable of hybridizing to the poly(A) stretch of the poly(A) nucleic acids wherein the capture probe is immobilized to a solid support;
wherein the hybridization composition comprises the sodium salt of the hybridization solution in a concentration selected from 37.5 mM to 125 mM, 50 mM to 100 mM and 62.5 mM to 87.5 mM and the tetraalkylammonium salt in a concentration selected from 0.125M to 1.25M, 0.25 M to 1 M and 0.375 M to 0.75 M,
and incubating said hybridization composition under conditions to form nucleic acid-hybrids between the poly(A) nucleic acids and the capture probe;
(b) separating the formed hybrids from the remaining sample.

10. The method according to one or more of claims 1 to 9, having one or more of the following characteristics:
a) the capture probe is a capture oligonucleotide comprising a single-stranded sequence complementary to the poly(A) stretch of the poly(A) nucleic acids;
b) the capture probe is an oligo(T)- or oligo(U)-comprising oligonucleotide;
c) the capture probe is bound to a solid support, wherein preferably, the solid support is provided by particles such as magnetic particles;
d) the hybridization composition is incubated at elevated temperature of 60°C or above and is subsequently incubated at a temperature of 40°C or below, preferably at room temperature to allow hybridization of the poly(A) RNA to the capture probe;
e) the method comprises the following additional steps:
(c) optionally washing the separated hybrids and
(d) releasing poly(A) nucleic acids from the washed hybrids;
f) the hybridization composition comprises a detergent and/or a chelating agent;
g) the sodium salt is a non-chaotropic salt;
h) a single poly(A) nucleic acid isolation cycle is performed to isolate the poly(A) nucleic acids; and/or
i) the isolated poly(A) nucleic acid is poly(A) RNA and wherein the method comprises sequencing the isolated poly(A) RNA, wherein preferably, sequencing is performed by next generation sequencing.

11. A method for sequencing poly(A) nucleic acids, preferably poly(A) RNA, comprising:
(a) isolating poly(A) nucleic acids from a nucleic acid containing sample using the method according to one or more of claims 1 to 10;
(b) sequencing the isolated poly(A) nucleic acid molecules.

12. An aqueous hybridization solution suitable for hybridizing poly(A) nucleic acids to a capture probe which is capable of hybridizing to the poly(A) stretch of the poly(A) nucleic acids, comprising:
aa. a sodium salt in a concentration of ≤ 500 mM;
bb. a quaternary ammonium salt.

13. The aqueous hybridization solution according to claim 12, having one or more of the following characteristics:
i) the hybridization solution comprises the sodium salt in a concentration selected from 50 mM to 500 mM, 75 mM to 400 mM, 85 mM to 350 mM, 100 mM to 300 mM, 115 mM to 250 mM, 120 mM to 225 mM and 125 mM to 200 mM;
ii) the hybridization solution comprises the quaternary ammonium salt in a concentration ≤ 3 M, preferably in a concentration selected from 0.25 M to 3 M, 0.5 M to 2.5 M, 0.75 M to 2 M and 0.75 M to 1.5 M;
iii) the sodium salt is sodium chloride;
iv) the quaternary ammonium salt is a tetraalkylammonium salt, preferably selected from tetramethylammonium salts (TMA) and tetraethylammonium salts (TEA);
v) the hybridization solution comprises:
aa. a sodium salt, preferably sodium chloride, in a concentration selected from 50 mM to 350 mM, 100 mM to 300 mM, 115 mM to 250 mM, 120 mM to 225 mM and 125 mM to 200 mM; and
bb. a tetraalkylammonium salt, preferably selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB), as quaternary ammonium salt in a concentration selected from 0.25 M to 3 M, 0.5 M to 2.5 M, 0.75 M to 2 M and 0.75 M to 1.5 M;
vi) the hybridization solution comprises:
aa. a sodium salt which is sodium chloride in a concentration selected from 75 mM to 250 mM, 100 mM to 200 mM and 125mM to 175mM;
bb. a tetraalkylammonium salt selected from the group consisting of tetraethylammonium chloride (TEAC), tetramethylammonium chloride (TMAC), tetramethylammonium nitrate (TMAN), tetraethylammonium bromide (TEAB) and tetramethylammonium bromide (TMAB) as quaternary ammonium salt in a concentration selected from from 0.25 M to 3 M, 0.5 M to 2.5 M, 0.75 M to 2 M and 0.75 M to 1.5 M.

14. A kit for isolating poly(A) nucleic acids from a nucleic acid containing sample, comprising:
(a) a hybridization solution according to claim 12 or 13;
(b) a capture probe capable of hybridizing to the poly(A) stretch of the poly(A) nucleic acid.

15. A method for isolating poly(A) nucleic acids having a single stranded poly(A) stretch from a nucleic acid containing sample comprising:
(a) hybridizing poly(A) nucleic acids to a capture probe capable of hybridizing to the poly(A) stretch of the poly(A) nucleic acids to form nucleic acid-hybrids between the poly(A) nucleic acids and the capture probe;
(b) separating the formed hybrids from the remaining sample;
(c) washing the separated hybrids with a hybridization solution according to claim 13 or 14, wherein the components of the hybridization solution can be added as single solution to the hybrids or may be added separately in any order to the hybrids to generate the hybridization solution used for washing;
(d) releasing poly(A) nucleic acids from the washed hybrids.

16. The method according to claim 15, wherein a washing composition is established due to the addition of the hybridization solution and optionally a dilution solution wherein said washing composition has one or more of the following characteristics:
a) the washing composition comprises the sodium salt of the hybridization solution in a concentration ≤ 250 mM, preferably in a concentration selected from 25 mM to 250 mM, 35 mM to 200 mM, 40 mM to 175 mM, 50 mM to 150 mM, 55 mM to 125 mM, 60 mM to 115 mM and 60 mM to 100 mM; and/or
b) the washing composition comprises the tetraalkylammonium salt of the hybridization solution in a concentration ≤ 1.5 M, preferably in a concentration selected from 0.125 M to 1.5 M, 0.25 M to 1.25 M, 0.375 M to 1 M and 0.375 M to 0.75 M; and/or
c) the washing composition comprises the sodium salt of the hybridization solution in a concentration selected from 25 mM to 175 mM, 37.5 mM to 150 mM, 50 mM to 125 mM and 62.5 mM to 100 mM and the tetraalkylammonium salt in a concentration selected from 0.125 M to 1.5 M, 0.25M to 1.25M, 0.375 M to 1 M and 0.375 M to 0.75 M; and/or
d) the washing composition comprises the sodium salt of the hybridization solution in a concentration selected from 37.5 mM to 125 mM, 50 mM to 100 mM and 60 mM to 100 mM and the tetraalkylammonium salt in a concentration selected from 0.125 M to 1.5 M, 0.25M to 1.25M, 0.375 M to 1 M and 0.375 M to 0.75 M.
